# EUROPEAN PATENT APPLICATION

(11) **EP 3 517 143 A1**
(43) Date of publication of application: **31.07.2019**
(21) Application number: 19153720.8
(22) Date of filing: 25.01.2019
(51) Int. Cl.: A61L 15/42, A61L 15/56

(54) **WETNESS INDICATOR WITH HARDENERS AND CRYSTALLIZERS**

(30) Priority: 25.01.2018 US 201862621781 P
(71) Applicant: The Procter & Gamble Company, Cincinnati, OH 45202 (US)
(72) Inventor: Kutay, Benjamin John, Cincinnati, Ohío 45202 (US); Klofta, Thomas James, Cincinnati, Ohio 45249 (US); Corzani, Italo, 66100 Chieti (IT)
(74) Representative: Kremer, Véronique Marie Joséphine

(57) **Abstract**

An article for baby care or feminine care comprising a wetness indicator comprising at least one colorant, at least one stabilizer, and from 0.1% to 70% by weight of a hardening agent and a crystallizing agent, and wherein the wetness indicator has at least three of five parameters ranges for hot to cold solidification rate, cold to hot melting rate, set point, melt point, and infinite shear rate.

## Description

### FIELD OF INVENTION

Disclosed are wetness indicator formulations that comprise hardeners, crystallizers, colorants and stabilizers.

### BACKGROUND OF THE INVENTION

Many disposable absorbent articles comprise a wetness indicator. Most wetness indicator compositions may comprise a colorant adapted to change in appearance, i.e., appear, disappear, change color, etc., upon contact with liquids such as urine, runny bowel movements, menses, etc., in the absorbent article. The colorant or color changing active used in many wetness indicator compositions are pH indicators. However, current pH-based wetness indicators may be unreliable, having issues with processability, premature triggering during storage, and/or colorant leaching issues, plus there are limits as to the variety of beginning and final color options. Therefore, there is a continuing need for simple wetness/fluid indicators that can provide a variety of color options and a continuing need for ways to improve the processability and stability of such wetness indicators within the absorbent articles.

### SUMMARY OF THE INVENTION

An absorbent article for baby care or feminine care is provided comprising a wetness indicator, the wetness indicator comprising at least one colorant, at least one stabilizer, and from about 0.1% to about 70% by weight of at least one of a hardening agent and crystallizing agent, wherein the wetness indicator has at least three of the parameters consisting of:
(i) a hot to cold solidification rate of Delta(G')/Delta(°C) from about 3,800 to about 27,000 Pa/°C ;
(ii) a set point temperature from about 60°C to about 110°C;
(iii) an infinite shear rate from about 0.02 to about 0.5 sec⁻¹;
(iv) a cold to hot melting rate from about 3,700 to about 11,000 Pa/°C ; and
(v) a melt point temperature from about 58°C to about 135 °C.

### BRIEF DESCRIPTION OF THE DRAWINGS

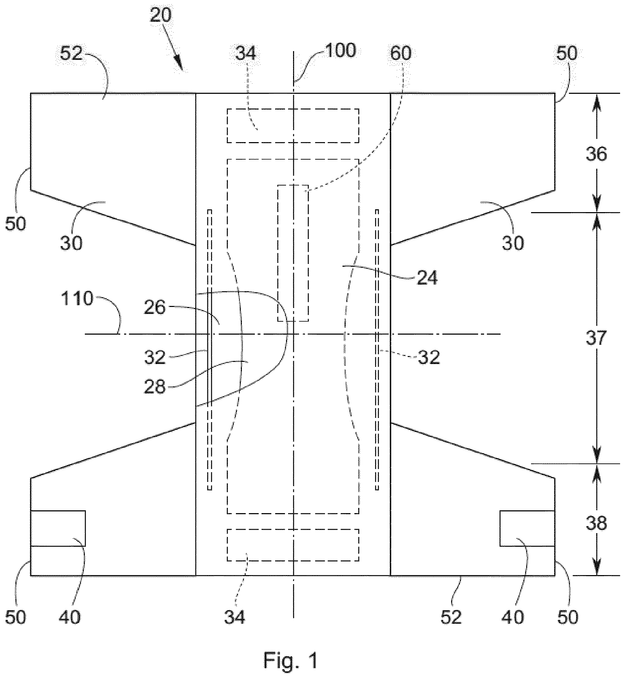

Fig. 1 is a top view of an absorbent article according to an aspect of the invention.

### DETAILED DESCRIPTION OF THE INVENTION

### Definitions

"Absorbent article" refers to devices which absorb and contain liquids. In some embodiments, absorbent article may refer to devices that absorb body exudates and, more specifically, may refer to devices which are placed against or in proximity to the body of the wearer to absorb and contain the various exudates or body fluids discharged from the body. Absorbent articles may include, but are not limited to, diapers, training pants, adult incontinence undergarments, feminine hygiene products, breast pads, bibs, and the like. As used herein, the term "body fluids" or "body exudates" includes, but is not limited to, urine, blood, vaginal discharges, breast milk, sweat and fecal matter.

"Article for baby care or feminine care" means products and/or methods relating to disposable absorbent and/or non-absorbent articles including adult incontinence garments, bibs, diapers, training pants, infant and toddler care wipes; catamenial pads, incontinence pads, interlabial pads, panty liners, pessaries, sanitary napkins, tampons and tampon applicators, and/or wipes.
As used herein, the term "colorant" refers to any dye, ink, pigment, inks that comprise dyes or pigments, pH indicators, metal indicators, oxidation or reduction indicators, solvatochromic colorants, biological colorant indicators that change color upon contact with a biological component of an exudates, food dyes or pigments, natural dyes or pigments, or any material that has the effect of changing its color or the color of its environment, or any combination thereof.

As used herein, the term "permanent colorant" refers to a colorant that maintains its color independent of environmental factors or one that does not change its color under most circumstance, such as a pH change or exposure to a liquid or specific components of the liquid, high humidities, or high or low temperatures, or even potential high pressures within the package.

"Absorbent core" means a structure typically disposed between a topsheet and backsheet of an absorbent article for absorbing and containing liquid received by the absorbent article and may comprise one or more substrates, an absorbent polymer material disposed on the one or more substrates, and a thermoplastic composition on the absorbent particulate polymer material and at least a portion of the one or more substrates for immobilizing the absorbent particulate polymer material on the one or more substrates.

"Comprise," "comprising," and "comprises" are open ended terms, each specifies the presence of what follows, e.g., a component, but does not preclude the presence of other features, e.g., elements, steps, components known in the art, or disclosed herein.

"Consisting essentially of' is used herein to limit the scope of subject matter, such as that in a claim, to the specified materials or steps and those that do not materially affect the basic and novel characteristics of the subject matter.

"Diaper" refers to an absorbent article generally worn by infants and incontinent persons about the lower torso so as to encircle the waist and legs of the wearer and that is specifically adapted to receive and contain urinary and fecal waste. As used herein, term "diaper" also includes "pants" which is defined below.

A "nonwoven" is a manufactured sheet, web, or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant" or "training pant", as used herein, refer to disposable garments having a waist opening and leg openings designed for infant or adult wearers. A pant may be placed in position on the wearer by inserting the wearer's legs into the leg openings and sliding the pant into position about a wearer's lower torso. A pant may be formed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).

One consideration for a wetness indicator is its processability, or its ability to easily and quickly be made and applied to an absorbent article. Ideally, a hot melt wetness indicator sets up, or changes from a hot melt into a solid, in a time quick enough to stay in the place it is placed, but not too fast, before it can spread and penetrate slightly into the material it is placed on. The wetness indicators of the present invention have a set point and a hot to cold solidification rate that allow high-temperature application to the article, which allows for better control.

Many wetness indicators comprise a colorant that is a pH indicator, that is, a material that changes color when a pH change occurs. This mechanism of color change where the pH controls the hue of the color is called halochromism. This color change based on pH is most typically used for urine indicators employed in diapers. For such halochromic colorants, the negative logarithm of its acid dissociation constant, or pKa, can be a way to measure or predict at what pH the material's color will change when contacted by aqueous fluids like pure water or urine or other aqueous based solutions. Typically, most diaper wetness indicator compositions employ pH indicator colorants that possess pKa values that are acidic and below a value of 7. In addition, many wetness indicator compositions on the market are intentionally formulated not to contain water within the composition. Finally, being neutral in charge allows the free acid forms of many pH indicator colorants like bromocresol green to be more easily formulated into an organic ingredient matrix which does not contain water.

An example of a halochromic colorant is the free acid form of bromocresol green which has a pKa value around 4.6. Thus, for an aqueous solution of bromocresol green, if one were to maintain a solution pH below 4.6, over 50% of the bromocresol green molecules would be protonated and yellow in color. If one were to raise the bromocresol green solution pH above 4.6, over 50% of the molecules would be in their blue-green anionic and conjugate base state. The solubility of the bromocresol green would also be higher in water at a pH above 4.6 since most of the molecules would be in their more water soluble charged anionic state. If one were to maintain a pH of 4.6, the color of this aqueous solution would be the result of combining 50% of the yellow molecules with 50% of the blue-green anionic molecules. For wetness indicator compositions possessing acidic pKa values below 7 like bromocresol green, the pH indicator colorant within the wetness indicator composition will be acidified in the dry state composition so it is maintained in its free acid form. For bromocresol green, its free acid form color is yellow and this neutral free acid form is more easily formulated into the organic wetness indicator composition which most typically do not contain any water. Within the wetness indicator composition containing bromocresol green, the neutral and protonated yellow acid form is stabilized by the addition of soluble acidic materials. Some pH indicator colorants possess a desirable yellow color when they are acidified to a pH below their pKa values. Some examples include bromocresol purple, bromocresol green and bromophenol blue which are all various shades of yellow in their free acid forms when they are acidified and protonated below their pKa values. Many of the acids used to maintain the free acid form of the pH colorant in the organic wetness indicator composition contain acid moieties like carboxylic acid groups or phosphate acid groups or sulfonic acid group and other acidic moieties. The acids most typically possess pKa values lower than the colorants to keep them in their protonated form. As noted below, these acids are also called acid stabilizers.

A pH indicator colorant used in a wetness indicator may or may not be stabilized with a stabilizer such as an acid stabilizer. The function of the acid stabilizer is to maintain the desired dry state acidic color of the colorant within the wetness indicator composition until it is insulted with a higher pH body fluid like urine. Thus, a good performing acid stabilizer will even maintain the desired dry state acidic color of the colorant within the wetness indicator after the diaper or diapers within the package are stored at high temperatures and humidities. For pH indicator colorants with pKa values below 7, the acid stabilizer helps to insure the pH indicator remains in its acidic and first color acidified dry state. This low pH color state of the colorant is formed because the acid stabilizer is more acidic and has a lower pKa than the colorant. If the pKa of the acid stabilizer is lower than the pKa of the pH indicator colorant, the stabilizer is more acidic than the pH indicator colorant. Certain pH indicator colorants may have multiple pKa values since the molecule possesses multiple acidic protons. For these colorants with multiple pKa values, all of the acid moieties will remain protonated as long as one of the acid stabilizers is more acidic and possesses a lower pKa than any of the pKa's of the colorant. In addition to possessing a lower pKa value, the amount of the acid stabilizer must be high enough to keep the pH indicator colorant completely protonated. If the number of higher concentration acid stabilizer molecules is lower than that of the pH indicator colorant, not all of the colorant's acid moieties will be protonated and stabilized.

Both the lower pKa and higher concentration of the acid stabilizer versus the pH indicator colorant insures the colorant stays in its acidic dry color state within the dry diaper until a color change is triggered by the higher pH of the urine (and/or other bodily exudates) which has a higher pKa than either the stabilizer or colorant. It is important to optimize the amount of acid stabilizer in the composition as too much can keep the pH colorant protonated for too long of time. The rise in pH above the pKa's for both the stabilizer and colorant is the result of contact with the higher pH of the urine which has a pKa higher than both the colorant and the acid stabilizer. Since the pKa of the urine is higher than both the colorant and acid stabilizer, the conjugate base and anionic forms of each can be formed. For bromocresol green, its anionic conjugate base form is blue-green in color. This conversion to the conjugate base form of the pH indicator colorant molecule results in a color change due to both bond rearrangement and conjugation changes within the colorant molecule. This anionic conjugate base form of the pH indicator colorant is also typically more ionic and more soluble in aqueous solutions like urine.

The aqueous urine with its higher pKa versus both the colorant and stabilizer also releases the proton from the acid stabilizer rendering it ineffective in maintaining the colorant in its free acid form. As noted, it is important to optimize the amount of acid stabilizer used in the composition since if too much acid is incorporated, both the intensity of the color change and the kinetics can be negatively impacted. With too much acid stabilizer, the vitality of the color change can be muted and the kinetics can be slowed. In other words, too much acid could lighten up the color change and also slow down the kinetics of this color change. Thus, it is important to optimize the amount of acid stabilizer within the composition so stability is maintained while also delivering fast kinetics so an attractive color vitality results from being wetted by an aqueous fluid like urine.

During use within a diaper, the wetness indicator is combined with, for example, urine, which has a pH of about 6. To be effective, the pKa's of both the acid stabilizer and colorant should be lower than this approximate pH value of 6 for the urine. As noted above, the acid stabilizer should also have a pKa lower than the pKa of the pH colorant in order to effectively keep it protonated within the dry state organic matrix prior to being wetting by an aqueous fluid like urine. After being wetted by the higher pH urine, both the pH indicator colorant and the acid stabilizer can be deprotonated so each converts into their anionic conjugate base forms. For bromocresol green, its conjugate base form is blue-green due to bond re-arrangement and light absorption changes. Thus, if the pKa of the pH colorant indicator is higher than the pH of the contacting urine, no color change will occur since the pH of the urine is acidic enough to maintain the free acid and protonated form of the pH colorant indicator. If the pKa of the pH colorant indicator is equal to the pH of the contacting urine, approximately half of the colorant molecules will be in the free acid form and the other half will be in the conjugate base form. This results in a color that is a blend of the free acid color state and its conjugate base color. For a dramatic and high contrast color change, the pH of the urine must be at least one unit, and preferable two units, above the pKa of the colorant to cause a visible color change. The concentration of both the acid stabilizer and colorant must also be optimized to lead to highly visible and fast forming color changes.

As noted, the concentration of the acid stabilizer can play a role in the color change kinetics. The acid stabilizer functions to keep the colorant in its dry state acid form since the stabilizer is more acidic and has a lower pKa than the pH indicator colorant. To keep the colorant acidified in its dry state color, the acid stabilizer must have a pKa lower than the pKa of the colorant. If the wetness indicator composition possesses a very high concentration of the acid stabilizer, the urine may not be able to dissociate and solvate all of the acidic protons from both the colorant and the stabilizer. Thus, no color change or only a faint color change may occur after the wetness indicator is contacted with the higher pH urine. This occurs because there is too high of a concentration of the acid stabilizer so excess protons exist in the environment of the pH colorant. Thus, many of the molecules of the pH colorant are maintained in their neutral free acid form. Thus, one needs to optimize both the acidity of the stabilizer as characterized by its pKa along with the concentration of the stabilizer.

While known wetness indicators may function sufficiently, the color options that are available in such systems are limited due to cost, formulation stability and processability, consumer color preferences, safety and purity constraints Thus, there is a continuing need for wetness indicators with a variety of color options for both the first and second color states. Even third and fourth color states can be possible for well-designed wetness indicator compositions.

Even though multiple color options are possible, it is imperative that the dry state color of the wetness indicator is stable during various storage and shipping scenarios that can occur from the plant where the diaper is manufactured to the ultimate placement of the diaper on a baby. For example, the color of the wetness indicator must be stable after a consumer might store the diaper, or package of diapers, within a hot and humid environment. The dry state color of the wetness indicator must be stable to other components within the diaper; especially those that are alkaline and can migrate to contact and possibly pretrigger the wetness indicator composition. In order to maintain the dry state color form, it is important to optimize both the amount and pKa of the acid stabilizer.

Interestingly, it has also been found that the hardness and crystallinity of components within the wetness indicator composition can influence the stability of the composition. Thus, both hardeners and crystallizers can contribute to the improved dry state color of the wetness indicator composition. It has been found that harder wetness indicator compositions can be more stable and more resistant to potential pretriggerants within the diaper. And crystallizers may speed up the solidification of the wetness indicator composition within the diaper during processing, thus inhibiting the migration of the wetness indicator composition to regions where pretriggerants may reside.

The "pretriggerant" is defined as any material or outside physical event that can cause the desired dry state color to change in color. For example, the physical property of high temperatures can act as a pretriggerant and cause the dry state color of poorly stabilized wetness indicators to change color due to oxidation. In some cases, high humidities can act as a pretriggerant to cause the dry state color to prematurely change to its wet state color. Within the diaper, materials like absorbent gelling materials, fillers like TiO₂ and calcium carbonate, alkaline surfactants, film and nonwoven materials, and even some adhesives that can contact the wetness indicator can also act as pretriggerants to undesirably change the dry state color of the wetness indicator.

For example, the pretriggerant could change the wetness indicator's dry state color of yellow to its wet state color of blue-green even before being contacted by a fluid like baby's urine. Or a pretriggerant like high temperatures might oxidize the dry state yellow color to an undesirable dark orange color.

Most typically for colorants stabilized with acid stabilizers, these pretriggerants have pKa values higher than both the acid stabilizer and colorant so their protons can be released. Not to be bound by theory, but it is hypothesized that a harder wetness indicator composition can resist deformation by other materials like pretriggerants in contact with the wetness indicator and especially those materials that may have higher pKa's than the colorant. Today, many diaper manufacturers pack their diapers under high pressures to maximimize the number of diapers within the package in order to reduce material and shipping costs. Because of these higher pressures, there is more intimacy between the diapers and all of the materials within a given diaper. Hard materials that possess pKa values higher than both the acid stabilizer and colorant can be especially detrimental because their hard nature allows easier penetration into softer materials like adhesives and wetness indicators. In addition, the higher pKa's of the hard pretriggerants could convert the colorant to its wet state color of its conjugate base. Thus, harder components that could cause pretriggering of the wetness indicator, especially if under presuure, include absorbent gelling materials, titanium dioxide, zinc oxide, and calcium carbonate. Thus, even though the hard absorbent gelling material may possess a pKa in the vicinity or higher than the pKa of the pH indicator colorant, if its penetration into the wetness indicator composition is hindered due to the high hardness of the wetness indicator composition, the dry state stability of the wetness indicator (WI) composition can be enhanced. Thus, the best stability performance for wetness indicator compositions with pH colorants with pKa values below 7 is observed when a hard wetness indicator composition is combined with the optimum concentration of an acid stabilizer which possesses a pKa lower than the pKa of the pH colorant while also applying the wetness indicator composition under conditions where it solidifies quickly upon the material to which it is applied.

The hardener or hardening agent may be defined as a material formulated within the wetness indicator composition in order to reduce the deformation that might be caused by another material within the diaper where this material may or may not be under pressure or in environments of high temperatures and humidities. As defined in the 10^{th} edition of The Condensed Chemical Dictionary (as revised by Gessner G. Hawley), hardness is the resistance of a material to deformation of an indenter of specific size and shape under a known load. Thus, a harder wetness indicator composition can resist deformation from high pKa materials like absorbent gelling materials that are under pressure and within close proximity to the wetness indicator.

Many hardeners are also effective crystallizers due to their linear molecular structure which can speed up the nucleation and ultimate solidification of the composition. For improved wetness indicator stability, it is important for the wetness indicator composition to harden and solidify as quickly as possible upon the substrate so it has limited chance of migrating to other regions of the diaper where pretriggerants might be present. Thus, it is optimum to use hardeners that crystallize quickly to prevent migration of the wetness indicator as it cools upon the substrate immediately after application. Here, crystallizers (or crystallizing agents) are defined as those ingredients that speed up the nucleation and solidification of the wetness indicator after application to a material within the diaper. Most typically, the wetness indicator composition is melted into its molten liquid state since most wetness indicator application systems are designed to apply hot and molten liquids. But, the various performance features of the wetness indicator are most effectively communicated to the consumer when they are in the solid state near or on the backsheet of the diaper. One of these performance features is stability, where the consumer expects the wetness indicator to possess the correct and consistent dry state color along with the expected color change after the baby urinates within the diaper. Caregivers also expect the color difference between the dry state and wet states to be suitably different and attractive so it is easy to detect a wetness event within the diaper.

For processing of the wetness indicator, if the time period between its liquid state at the applicator and the solid state on the diaper is shortened, a more stable wetness indicator diaper is made. Not to be bound by theory, but the crystallizer can speed up the nucleation and hot to cold solidification rate since the linear and more ordered crystallizer molecules can line up with one another more quickly to form a harder solid composition within the diaper. Most typically, the hot and molten wetness indicator is applied to the inside of the diaper's backsheet film. Upon contact with the cooler backsheet material, the wetness indicator undergoes a rapid decrease in the entropy of the system due to ordered crystal formation within the wetness indicator composition. This also leads to a concomitant enthalpy gain due to formation of these closely packed and ordered crystal structures within the wetness indicator. Molecules that are most likely to crystallize into ordered and tightly packed structures upon cooling are most typically linear and higher melting in nature since they form nucleation sites within the composition which speeds up the crystallization of the molten composition. These crystallizers also contribute to hardening the solid wetness indicator composition.

A measurement technique used to measure this hot to cold solidification rate employs a stress controlled rheometer instrument to measure the Delta(G')/Delta(°C) which is calculated by dividing DeltaG' (the change in G'in units of Pascals) by Delta°C (the change in temperature in units of degrees Celsius). This measurement is called the hot to cold solidification rate since the wetness indicator is first heated well above its melt point and then slowly cooled down on the sampling area of the rheometer. The details of this hot to cold solidification rate measurement are noted below. Here, G' is the storage modulus of the wetness indicator composition as measured in units of Pascals (Pa) and °C is the temperature in units of degrees Celsius. In this rheological technique to measure Delta(G')/Delta(°C), the wetness indicator composition is first heated up to its liquid and molten state and then allowed to cool down at a controlled rate (see method details below). Since the wetness indicator composition is first heated to its molten liquid state and allowed to cool, this measurement is specifically termed the "hot to cold solidification rate of Delta(G')/Delta(°C)." As noted, this value of Delta(G')/Delta(°C) correlates with the wetness indicator's solidification rate and it is optimum for it to be high so it can solidify quickly to avoid migration on or into neighboring materials of the diaper. High Delta(G')/Delta(°C) values will also correlate with sharper and more uniform wetness indicator patterns such that the edges of the rectangular pattern remain sharp and the corners do not become rounded. A high Delta(G')/Delta(°C) also aids in maintaining a uniform thickness of the wetness indicator composition within the diaper to allow for uniform dry and wet state colors along with uniform color changes after being contacted by a body fluid like urine. This uniform thickness also insures uniform stability of the wetness indicator composition. In one embodiment, the hot to cold solidification rate of Delta(G')/Delta(°C) may be from about 3,800 to about 27,000 Pa/°C. In some embodiments, the hot to cold solidification rate may be from about 4,600 to about 21,400 Pa/°C.

One example of a crystallizer is a paraffin wax which is made up of normal and saturated straight-chain or long-chain alkane hydrocarbons ranging in carbon lengths most typically from C18H38 to C32H66, although this range can vary due to source variations of the wax along with the particular refining process used for the wax clean up and purification. For synthetic paraffin waxes, the synthesis procedure will also affect the resultant chain length. Due to the linear structure of the saturated normal alkanes within most paraffin waxes, the straight-chain molecules can pack in close proximity with one another due to the high van der Waals forces that exist between their long and linear carbon chain. As noted, hard and crystalline compositions most readily result when linear and high melting ingredients like paraffin waxes are incorporated. Examples of paraffin waxes include The International Group's (Titusville, PA) IGI-1230A, IGI-1250A, and IGI-1260A. Shell Wax 200 and 400. Paraffin waxes like "Paraffin 150/160" from the Frank B. Ross Company (Rahway, NJ) would also function as crystallizers and hardeners. Other linear and crystalline waxes that function well as crystallization agents include linear polyethylenes like the Performalene™ M waxes (M70 wax, M80 wax, and M90 wax) from Baker Hughes Inc, and their Performalene™ polyethylene waxes like Performalene™ 400 (melting point of 84C) and Performalene™ 655 with a melting point of 100C. Among crystalline polyolefin waxes, those containing also acidic groups, like oxidized or maleated waxes or waxes derived from montanic acid, can be conveniently used in the present formulations. Linear primary and fully saturated alcohols also function well as crystallization agents and these include stearyl alcohol, behenyl alcohol and higher molecular weight primary alcohols with an INCI name of C20-40 Alcohols and possessing the trade name of Performacol™ 350 and Performacol™ 425 from Baker Hughes Inc.Another one of these crystallizing alcohols sold by Baker Hughes has an INCI name of C30-50 Alcohols and is sold under the trade name of Performacol™ 550. The International Group also sells some of these higher molecular weight and linear saturated primary alcohols as Acculinol™ line of alcohols. Other appropriate crystallizers include linear primary carboxylic acids like palmitic acid, stearic acid, behenic acid, or the higher melting point linear primary carboxylic acids trademarked as Unicid™ from Baker Hughes Inc. and Accucid™ line of higher molecular weight and linear primary carboxylic acids from the International Group. These higher molecular weight linear primary carboxylic acids include the Unicid™ 350 (melting point of 92C), Unicid™ 425 and Unicid™ 550 from Baker Hughes Inc. Given their acidic nature combined with their high crystallinity and surface hardness, the above mentioned linear primary carboxylic acids may function at the same time as acid stabilizers, as well as hardeners and crystallizers. Among non-acidic hardeners and crystallizers, also aliphatic polyesters may be usefully employed in the described wetness indicator formulations.

A wetness indicator may comprise from about 0.1% to about 70% by weight of a crystallizing agent (crystallizer), which may be one crystallizer or a combination of crystallizers disclosed herein. In some embodiments, the amount of crystallizing agent in the wetness indicator may be at least about 5%, at least about 10%, at least about 20%, at least about 25%, at least about 30%, at least about 40%, at least about 50%, or even at least about 60% by weight. In some embodiments, the crystallizing agent may be from about 20% to about 70%, from about 20% to about 40%, from about 25% to about 50%, from about 25% to about 60%, from about 25% to about 70%, from about 30% to about 70%, from about 30% to about 60%, from about 40% to about 70%, from about 40% to about 60%, from about 50% to 70%, from about 55% to 70%, from about 60% to about 70% by weight of the wetness indicator composition.

The hardness of the wetness indicator compositions of the present invention can be measured for example by the so called "Needle-Penetration" method, as described in ASTM D1321-04. The formulations of the present invention have a Needle penetration no greater than about 40 dmm at 23 °C and preferably no greater than about 150 dmm at 55 °C

A wetness indicator may comprise from about 0.1% to about 70% by weight of a hardener (hardening agent), which may be one hardener or a combination of hardeners disclosed herein. In some embodiments, the amount of hardener in the wetness indicator may be at least about 5%, at least about 10%, at least about 20%, at least about 25%, at least about 30%, at least about 40%, at least about 50%, or even at least about 60% by weight. In some embodiments, the hardener may be from about 20% to about 70%, from about 20% to about 40%, from about 25% to about 50%, from about 25% to about 60%, from about 25% to about 70%, from about 30% to about 70%, from about 30% to about 60%, from about 40% to about 70%, from about 40% to about 60%, from about 50% to 70%, from about 55% to 70%, from about 60% to about 70% by weight of the wetness indicator composition.

As noted above, compositions that set up and solidify quickly into hard compositions on the backsheet can lead to more stable compositions when pretriggerants might be present in the absorbent article. Most of these crystallizers and hardeners are linear in structure and upon shearing in the slot coating process for application to the diaper substrate, the wetness indicator composition can thin out under extreme shearing conditions. For the slot coating process, this thinning out, or reduction in viscosity, of the wetness indicator composition could lead to the following potential problems: 1) dripping off of the slot coater and contaminating other regions of the diaper or processing equipment, 2) poor pattern development of the wetness indicator on the substrate which could lead to unattractive patterns and heterogeneous thicknesses, 3) extreme shear thinning of the wetness indicator could also lead to problems with properly pumping the composition. For these reasons and others, an Infinite Shear Rate may be from about 0.02 to about 0.5 sec⁻¹. The details of obtaining this Infinite Shear Rate value are given below but basically, one uses a stress controlled rheometer and sets the temperature of the wetness indicator to that used in the processing equipment. Then, the viscosity of the composition is monitored while the shear rate is increased at a controlled rate to the desired maximum shear (usually several order of magnitude). At high shear rates, the composition will eventually show a sharp decrease in viscosity which continues to decrease as the shear continues to rise. For this data, one plots the viscosity in Pascals -seconds on the ordinate (Y-axis) of a two-dimensional graph and the shear rate on the abscissa (X-axis). One then curve fits a vertical line through the data points where the viscosity drops fast with increasing shear rates. The point at which this vertical line intersects the abscissa axis (X-axis) where the shear rate is plotted is the Infinite Shear Rate in units of sec⁻¹. In some embodiments, the infinite shear rate of the present invention's wetness indicators may be from about 0.02 to about 0.5 sec⁻¹, in other embodiments from about 0.05 to about 0.1 sec⁻¹.

For the reasons outlined above, the inclusion of crystallizers and hardeners can lead to improved stability of the wetness indicator within the diaper. But some hardeners and crystallizers possess excessively high melting points which can lead to excessively long times for the wetness indicator composition to melt in the tank or even worse, cause melting of the substrate polymer onto which the composition is coated. To better understand the melting behavior of the composition, a cold to hot melting rate can be measured for each wetness indicator composition. In this rheological measurement, the stress controlled rheometer is set up to measure the storage modulus, G',of the composition as it is heated up at a controlled rate from cooler starting temperatures (read below for details on this cold to hot melting rate measurement). As the temperature of the composition is heated up, the viscosity and storage modulus will eventually drop precipitously as it becomes totally liquified and melted with no inclusion of solid phase components. The slope of this sharply descending region of G' is termed the "cold to hot melting rate." By plotting the storage modulus G' in units of Pascals (Pa) on the ordinate (Y-axis) and the temperature in Celsius units (°C) on the abscissa (X-axis), one can calculate the slope by dividing the change in storage modulus, DeltaG', by the change in temperature, Delta°C, and arrive at the cold to hot melting rate ratio of Delta(G')/Delta(°C). The units of this cold to hot melting rate of Delta(G')/Delta(°C) are Pa/°C. In some embodiments, the cold to hot melting rate may be from about 3,700 to about 11,000 Pa/°C, and in some embodiments, from about 4,000 to about 7,300 Pa/°C. For the wetness indicating compositions according to the present invention, the melt point temperature (or melt temperature) is preferentially defined according to a rheological criterion i.e. as the temperature, in the field of temperatures above room temperature, per the following assessment. One needs to identify 2 consecutive sets (4 data points per set) of data points which are decreasing point to point by 5% or more in G', when that critieria is met, then choose the first point from the first set of points and use the temperature in Celsius as the melt point. In general this point is best determined, from a rheological method run from cold to hot via a controlled heating rate.

Alternatively one can use the point at which the Elastic Modulus G' and the Viscous Modulus G" cross, or also (which is equivalent by definition) the temperature at which Tan Delta is equal to 1. In fact at temperatures below said point, the Elastic Modulus G' (that expresses the "solid character" of the material) prevails over the Viscous Modulus (that on the contrary expresses the "fluidity" of the material) and therefore the material behaves like a solid.

On the contrary, at temperatures above said point the opposite is true: G" prevails over G' and therefore the material behaves like a fluid or a liquid.

From this measurement of the cold to hot melting rate, one can obtain data on how quickly the composition melts in the adhesive melt tank. During diaper line downtimes, the temperature of the wetness indicator melt tank will be turned down to prevent its potential degradation. Upon eventual startup, the temperature of the melt tank is raised to the appropriate set point temperature for optimum processing. To prevent excessive diaper line downtimes, it is important for the wetness indicator composition to melt as quickly as possible within its melt tank to maintain production targets of the diapers. This cold to hot melting rate can also indicate how quickly the wetness indicator composition might soften up under high temperature exposures. This is important to know in order to avoid softening within the diaper which could lead to wetness indicator migration to other regions of the diaper or even a degradation of the pattern sharpness.

Another important and fundamental characteristic of the wetness indicator composition is its melt point in units of degrees Celsius (°C). The melt point is important for the correct mixing of the wetness indicator composition during making to insure uniform homogeneity of the composition. A temperature a bit higher than the melt point should also be maintained during dispensing of the wetness indicator composition to insure both homogeneity and quick solidification. The melt point is also important for properly setting up the melt tank and slot coater application equipment in order to coat a quality pattern within the diaper and maintain stability within the equipment. In addition, the melt point is indicative of how stable the wetness indicator composition is during transport and storage along with its stability within the diaper. If the melt point is too high, the composition could melt and damage other materials within the diaper or take excessively long to first melt within its melt tank. If the melt point is too low, the wetness indicator composition could migrate throughout the diaper if the consumer inadvertently stores the product at high temperatures. In some embodiments, the melt point temperature may be from about 58°C to about 135°C, and in other embodiments froma bout 65°C to about 120°C.Along with crystallizers and hardeners, it is still important that the wetness indicator composition contains acid or basic stabilizers to maintain the colorant in its desired dry state color. Thus, both stabilization strategies contribute to providing a stable wetness indicator composition.

The wetness indicator compositions of the present invention can also include more than one colorant, wherein each colorant is stabilized in its first color state with its own stabilizer. Because the first colorant and the first stabilizer may have a similar pKa and the second colorant and the second stabilizer may have a similar pKa, each colorant in the wetness indicator may be maintained (or stabilized) in its first color state until triggered to its second color state by the urine or other exudate. In some cases, the colorants' pKa's may be low and can be stabilized with very acidic stabilizers. In other cases, the pKa's may be high and the colorants can be stabilized with basic stabilizers with high pKa values above 7. In any case, the use of customized stabilizers in the present invention can allow for a much greater variety of colorants that can be utilized in wetness indicator compositions. This use of various combinations of colorants and stabilizers results in a large variety of both dry state and wet state colors for the wetness indicator compositions. With optimum formulation design with multiple colorants and multiple stabilizers, one can even trigger different colors to appear at different times after a body fluid like urine contacts the wetness indicator composition. In addition, the selection of the correct stabilizer can lead to enhanced dry state stabilization of the desired dry state color. As noted, this chemical stabilization along with formulations that set up and solidify quickly as hard compositions on the absorbent article during application can lead to improved dry state stabilization before the diaper is used.

In some embodiments of the present invention, a wetness indicator comprises a first stabilizer where its pKa is either at most about one unit above or multiple units below the pKa of the first colorant, and a second stabilizer where its pKa is either at most about one unit above or multiple units below the pKa of the second colorant. As noted, even third and/or fourth colorants and third and/or fourth stabilizers can be incorporated. In some embodiments, the wetness indicator comprises a first and second colorant and a first and second stabilizer, wherein the pKa of the first stabilizer is from about two units below to about one unit above the pKa of the first colorant, and the pKa of the second stabilizer is from about two units below to about one unit above the pKa of the second colorant. In some embodiments, the pKa of the first stabilizer may be from about one unit below to about one unit above the pKa of the first colorant, and the pKa of the second stabilizer may be from about one unit below to about one unit above the pKa of the second colorant. In some embodiments, the pKa's of the two colorants (and stabilizers) may be close, but in other embodiments, the pKa's of the two colorants (and their respective stabilizers) may be identical or at most, about 4 to about 5 units apart. If the pKa's of the colorants are relatively close or identical to one another, it may be possible to stabilize the composition with a single stabilizer. Or where the colorants have widely separated pKa values, a single stabilizer can be used to create an interesting array of different colors to appear as a function of time after contact with a body fluid like urine. For compositions where the pKa's of the colorants are further apart, each colorant will most likely require its own specific stabilizer in order to combine them effectively into the wetness indicator composition. As the pKa's of the pH colorants become further apart, the time difference for each of them to change color upon wetting with a fluid like urine becomes longer. This can be advantageous if one wishes to create different colors at different points in time after the urine contacts the wetness indicator composition.

Currently, many diaper wetness indicators transition from a yellow dry state to a blue-green color after urine contacts the wetness indicator (WI) composition. This is due to the common choice of bromocresol green as the pH indicator colorant in various WI compositions. Bromocresol green is commonly used because its yellow to blue-green color change is well liked by care givers and its pKa of 4.6 is optimum for use in WI compositions. Also, its yellow free acid form is readily soluble in most lipophilic ingredients used in adhesive compositions. This pKa of 4.6 for bromocresol green is ideal since the yellow free acid state of bromocresol green can be stabilized in the dry state by the use of low cost chemicals functionalized with carboxylic acid groups since many molecules possessing carboxylic acid moieties possess pKa's similar or lower than the pKa of bromocresol green. Depending on the chemical structure of the particular carboxylic acid, one can expect its pKa to be in the range of 3 to 5 which is typically acidic enough to convert the bromocresol green colorant into its yellow free acid form. Even though carboxylic acid moieties are ideally suited for a colorant like bromocresol green, they may not be strong enough acids for other pH indicating colorants with pKa values lower and more acidic than bromocresol green. Thus, the acid stabilizer's pKa must be close, or preferably lower than the pKa of the pH indicating colorant in order to form the free acid colored state in the dry state within an absorbent article like a diaper. Preferably, the acid stabilizer is a stronger acid and possesses a lower pKa than the colorant in order to insure that it is completely protonated in its free acid color state. In addition, bromocresol green's pKa of 4.6 is much lower than the average pH of urine such that when wetted with urine, it quickly and efficiently changes to its blue-green color state as the proton is released from the bromocresol green and the conversion into the blue-green conjugate base state takes place. Thus, because its pKa is between the pKa of many carboxylic acid containing molecules and the pH of urine, bromocresol green is an optimum pH indicating colorant with attractive dry and wet state colors. Also, bromocresol green possesses an attractive color change of yellow in its acidic dry state to a blue-green color after it is converted to its conjugate base form after the more alkaline urine contacts the wetness indicator.

Some caregivers would prefer different or additional color choices within their wetness indicators. For example, a color change of orange in the dry state before the diaper is put on the baby and blue when the baby urinates within the diaper, or yellow in the dry state and purple in the wet state. Here, for this change of yellow to purple, bromocresol purple might be an ideal candidate with its known color change of yellow in its free acid form and purple when it is deprotonated to its conjugate base form. But, bromocresol purple has a higher pKa of 6.3 compared to the pKa of 4.8 for bromocresol green. So, although bromocresol purple's higher pKa allows it to be easily stabilized in its yellow dry state with chemicals functionalized with carboxylic acid moieties since they are much more acidic than the bromocresol purple, the bromocresol purple does not easily change to purple upon contact with urine since its pKa is higher than the average pH of baby's urine. This close proximity of the urine's pH to the pKa of the bromophenol purple results in slow kinetics for the color change of the bromocresol purple and it can take a very long time for it to fully develop a clearly visible dark purple color. And depending on the acidity of the wetness indicator composition, the bromocresol purple may remain protonated and never change to purple in its conjugate base form. To achieve the dark purple color of bromocresol purple, one would have to raise the pH one to two units above its pKa value of 6.3. This insures that the bromocresol purple is in its highly conjugated and purple conjugate base form. One might add an alkaline ingredient to the wetness indicator composition to increase the pH upon urine contact but this typically degrades and negatively affects the dry state stability of the yellow acidic color. Prior to use on one's baby, the alkaline additive could leach out of the wetness indicator composition, especially in humid environments, to increase the pH and convert the free acid into the purple conjugate base form. As noted, this dry state stability is especially challenging in humid environments where the moisture might solubilize and increase the activity of the added alkaline ingredient. In this case, the increased solubility of the alkaline ingredient could raise the pH above the pKa of the bromocresol purple and pretrigger its color change to purple in the dry state.

The present invention discloses that dry and wet state colors can be formulated if two pH colorants are combined into a single formulation. For example, a wetness indicator may comprise a first and second colorant and also a first and second stabilizer, where the first colorant and the first stabilizer have similar pKa's and the second colorant and second stabilizer have similar pKa's. As noted, the stabilizers maintain the desired dry state color of the colors when the wetness indicator compostion is subjected to severe environmental conditions like high humidities and temperatures or even pretriggerants within the diaper at could destabilize the wetness indicator. Finally, hard compostions that set up quickly during the application process also contribute in stabilizing the composition; especially if pretriggerant are in the diaper and in close proximity to the wetness indicator. In some embodiments, the first stabilizer's pKa is from about two units below to about one unit above the pKa of the first colorant, and the second stabilizer's pKa is from about two units below to about about one unit above the pKa of the second colorant. In some embodiments, the pKa of the colorant and stabilizer may be from about 1.5 to about 3.5, while the pKa of the second colorant and stabilizer may be from about 3.0 to about 5.0, in some embodiments from about 3.5 to about 5.5.

For example, the combination of two colorants such as phloxine B acid and the free acid of bromophenol blue can provide either a color change from yellow to purple or orange to purple. This can be accomplished by careful selection of the acid stabilizers for each of the colorants. For the yellow to purple color change, a phosphorous based stabilizer acid like cetyl phosphate has a pKa low enough to acidify both the phloxine with its pKa near 2.9 and the bromophenol blue with its pKa near 4.0. It should be noted that alkyl phosphate stabilizers like cetyl phosphate and stearyl phosphate and cetearyl phosphate can be complex mixtures of multiple molecules. Thus, a cetyl phosphate from a given supplier may contain traces of phosphoric acid, monocetyl phosphate, dicetyl phosphate and tricetyl phosphate. This combination can still be effective in acidifying the colorant because some or all of the trace materials may be more acidic than the colorant. For example, phosphoric acid has a very low pKa value and so a stabilizer containing traces of phosphoric acid can still be very effective in acidifying colorants within the wetness indicator matrix. Further, if the stabilizer is substantially one molecule, meaning at least about 90% one molecule, in some cases at least about 95% one molecule, or in some cases at least about 99% one molecule, the pKa of the stabilizer may be considered to be the pKa of the predominate molecule. Many acid and base stabilizers will be a mixture of multiple acid ingredients or a mixture of multiple base ingredients, and a key property for their proper functioning within the wetness indicator composition is to be either a stronger acid or stronger base respectively than the colorant they are stabilizing. At a pH below their pKa values, the phloxine is colorless and the bromophenol blue is yellow. If the pH is above their pKa values, the phloxine is red and the bromophenol blue is blue such that the mixture of red and blue results in a final purple color in the wet state. Thus, the resulting dry state color is yellow and the resulting wet state color after being insulted with urine is purple for this combination. But, only a low concentration of the phosphorous based acid stabilizer can be used since it is much more acidic than the bromophenol blue while being closer in acidity to the phloxine. If one includes too much of the cetyl phosphate acid stabilizer which may contain traces of phosphoric acid, the urine might not be able to solvate and deprotonate the acid stabilizer. In such a case, there could be enough remaining acidic protons to keep both the phloxine and bromophenol blue in their protonated acid states. Being a strong acid with a pKa(s) lower than both the pKa's of the phloxine and bromophenol blue, this cetyl phosphate acid stabilizer can stabilize both the phloxine and bromophenol blue into their free acid states. As noted, if too much phosphorous based acid is used, the yellow dry state is achieved but the color change to purple after wetting with urine is very slow and the color is faint. This is because the strong phosphorous based acid stabilizer hinders the rise in pH above the pKa of the bromophenol blue. Essentially, the system can be too acidic such that the formation of the conjugate bases of the colorants is hindered or takes too long of a time period after contact with the body fluid. To achieve the purple wet conjugate base state color with acceptable kinetics, a low level of the phosphorous based stabilizer acid like Clariant's Cetyl Phosphate (trade name of Hostaphat™ CC-100) is incorporated along with a carboxylic acid based ingredient for acidification of the bromophenol blue. For a wetness indicator composition containing both colorants of the free acid of phloxine and the free acid of bromophenol blue, an optimum amount of Hostaphat™ CC-100 stabilizer is around 0.5 to 1.5% by weight. This is equivalent to around 0.05% to 0.15% of elemental phosphorous being contributed from the acid stabilizer. Not being too strong of an acid stabilizer but possessing a pKa around that of the colorant, the carboxylic acid can keep the bromophenol blue colorant acidified in its yellow dry state but it does not hinder the quick color change to purple after wetting with urine for this particular combination of phloxine, bromophenol blue, and the two acid stabilizers. The carboxylic acid based stabilizer is strong enough to maintain the yellow dry state but not so strong as to hinder a rise in pH well above the pKa of the bromophenol blue after contact with baby's urine. The addition of the carboxylic acid based stabilizer also aids in maintaining the yellow dry color if the caregiver exposes the diaper to high humidities and temperatures. Finally and since it possesses a linear and fully saturated alkyl chain, the Hostaphat™ CC-100 can also function as a crystallizer although its effectiveness would be limited since low concentrations are typically used.

Example 1 is a wetness indicator composition that changes from a yellow dry state color to a bluish-green wet state color and it contains three acid stabilizers with Foral™ AX-E, Hostaphat™ CC-100 and Unicid™ 550. The acid stabilizer Unicid™ 550 also functions as a crystallizer and hardener agent. Thus, Example 1 possesses both chemical stability due to the inclusion of acid stabilizers and physical stabilization due to the inclusion of crystallizers, which allow the WI composition to set up quickly into a hard solid on the substrate. Its quick solidification on the substrate during manufacturing prevents it from migrating into other regions of the diaper where pretriggerants might be present. Example 1's hardness as a solid also inhibits penetration of pretriggerants into the WI while the acid stabilizers maintain the protonated dry state colors of the colorants even in hot and humid environments.

| Example 1 - Yellow to Blue-Green | W/W (%) | CAS No. | Function |
|---|---|---|---|
| Performathox 450 ethoxylate* | 10.00 | 251553-55-6 | Surfactant |
| Performathox 480 ethoxylate* | 20.00 | 251553-55-6 | Surfactant |
| Foral AX-E 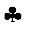 | 10.00 | 9005-00-9 | Tackifying Agent/Stabilizer |
| Unicid 550^{Ω} | 58.3 | 251554-90-2 &9002-88-4 | Crystallizer/Hardener/Stabilizer |
| Hostaphat™ CC-100^{>} | 0.2 | 3539-43-3 | Stabilizer |
| Irganox 1010 ^{□} | 1.0 | 1709-70-2 | Anti-Oxidant |
| Bromocresol Green Free Acid 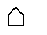 | 0.5 | 76-60-8 | Colorant |

| | | | |
|---|---|---|---|
| *Performathox 450 and Performathox 480 as supplied by Baker-Hughes of Houston, TX. 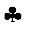 Foral AX-E as supplied by Eastman Chemicals of Kingsport, TN. ^{□}Irganox 1010 as supplied by BASF of Florham Park, NJ. ^{>}Hostaphat™ CC-100 as supplied by Clariant Inc. of Charlotte, NC "Unicid 550 as supplied by Baker-Hughes of Houston, TX. 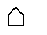 Bromophenol Green free acid as supplied by TCI Chemicals of Portland, OR. | | | |

For Example 1 above, the inclusion of the Unicid™ 550 which is a hard, crystalline, acidic substance that has a high melt of 101°C increases the se tpoint temperature of the wetness indicator composition. The set point temperature (°C) is a rheological property and calculated from a two-dimensional graph where the storage modulus (G') is graphed as Pascals (Pa) on the ordinate axis (Y-axis) and the temperature in degrees Celsius (°C) is plotted on the abscissa axis (X-axis). For the wetness indicating compositions according to the present invention, the set point temperature (or setting temperature) is preferentially defined according to a rheological criterion i.e. as the temperature, in the field of temperatures above room temperature, per the following assessment. One needs to identify 2 consecutive sets (4 data points per set) of data points which are increasing point to point by 5% or more in G', when that criteria is met, then choose the first point from the first set of points and use the temperature in Celsius as the set point. In general this point is best determined, from a rheological method run from hot to cold via a controlled heating rate. The set point temperature is that temperature where the G' begins to rise dramatically and is calculated as the inflection point from the two-dimensional graph of G' versus °C. The end point for the set point data will be determined when the set point criteria can no longer be met along the data sets that are experiencing the change in G'vs. the temperature decrease. The end point temperature will therefore be set by the last point from the last set of points that meet the set point criteria. Set point temperature is reflective of the temperature at which the wetness indicator solidifies and it is important for the processing temperature of the wetness indicator composition to be only about 1 to 15 °C higher than the set point temperature right before the composition contacts the cooler substrate temperature. Doing this allows the wetness indicator to set up quickly in order to avoid migration in other regions of the diaper. As noted, quick solidification also leads to sharper pattern formation and more uniform thickness development. Details on the measurement of the rheological setpoint temperature can be found below. In some embodiments, the set point temperature of the present wetness indicators may be from about 60°C to about 110°C.

Example 2 is a subtle modification of Example 1 with the addition of the ethylene-ethylene acrylic acid copolymer that functions as both an acid stabilizer and rheological modifier. Here in Example 2, the ethylene-ethylene acrylic acid from Honeywell raises the viscosity for improved processing during application while still setting up quickly to create a hard formula that is stable to most pretriggerants within the diaper and that also resists color changes when exposed to high temperatures and humidities. Example 2 is a wetness indicator composition that changes from a yellow dry state color to a bluish-green wet state color and it contains four acid stabilizers with Foral™ AX-E, Hostaphat™ CC-100, AC™-5120 from Honeywell Inc., and Unicid™ 550. As noted, the acid stabilizer Unicid™ 550 also functions as a crystallizer and hardener. Thus, this Example 2 composition possesses both chemical stability due to the inclusion of acid stabilizers and physical stabilization due to the inclusion of crystallizers and hardeners which allow the wetness indicator (WI) composition to set up quickly into a hard solid on the substrate. Its quick solidification on the substrate during manufacturing prevents it from migrating into other regions of the diaper where pretriggerants might be present. Example 2's hardness as a solid also inhibits penetration of pretriggerants into the WI while the acid stabilizers maintain the protonated dry state colors of the colorants even in hot and humid environments. Here is the Example 2 composition which exemplifies not only chemical stabilization but also physical stabilization via the inclusion of hardeners and crystallizers:

| Example 2 - Yellow to Blue-Green | W/W (%) | CAS No. | Function |
|---|---|---|---|
| Performathox 450 ethoxylate* | 10.00 | 251553-55-6 | Surfactant |
| Performathox 480 ethoxylate* | 20.00 | 251553-55-6 | Surfactant |
| Foral AX-E 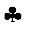 | 10.00 | 9005-00-9 | Tackifying Agent/Stabilizer |
| Ethylene Acrylic Acid AC-5120^{Ω} | 20.0 | 9010-77-9&79-10-7 | Stabilizer & Binding Agent |
| Unicid 550^{Ω} | 38.3 | 251554-90-2&9002-88-4 | Crystallizer/Hardener/Stabilizer |
| Hostaphat™ CC-100^{>} | 0.2 | 3539-43-3 | Stabilizer |
| Irganox 1010 ^{□} | 1.0 | 1709-70-2 | Anti-Oxidant |
| Bromocresol Green Free Acid 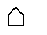 | 0.5 | 76-60-8 | Colorant |

| | | | |
|---|---|---|---|
| *Performathox 450 and Performathox 480 as supplied by Baker-Hughes of Houston, TX. 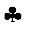 Foral AX-E as supplied by Eastman Chemicals of Kingsport, TN. ^{□}Irganox 1010 as supplied by BASF of Florham Park, NJ. ^{>}Hostaphat CC-100 as supplied by Clariant Inc. of Charlotte, NC. ^{Ω}Ethylene Acrylic Acid as supplied as AC-5120 by Honeywell Inc. of Morristown, NJ. ^{Ω}Unicid 550 as supplied by Baker-Hughes of Houston, TX. 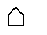 Bromophcnol Green free acid as supplied by TCI Chemicals of Portland, OR. | | | |

Example 3 is a subtle modification of Example 2 with the use of Unicid 350 instead of Unicid 550. Here, Example 3 is another wetness indicator composition that changes from a yellow dry state color to a bluish-green wet state color and it contains four acid stabilizers with Foral™ AX-E, Hostaphat™ CC-100, AC™-5120 from Honeywell Inc., and Unicid™ 350. As noted, the acid stabilizer Unicid™ 350 also functions as a crystallizer and hardener. Thus, this Example 3 composition possesses both chemical stability due to the inclusion of acid stabilizers and physical stabilization due to the inclusion of crystallizers and hardeners which allow the WI composition to set up quickly into a hard solid on the substrate. Its quick solidification on the substrate during manufacturing prevents it from migrating into other regions of the diaper where pretriggerants might be present. Example 3's hardness as a solid also inhibits penetration of pretriggerants into the WI while the acid stabilizers maintain the protonated dry state colors of the colorants even in hot and humid environments. Here is the Example 3 composition which exemplifies not only chemical stabilization but also physical stabilization via the inclusion of hardeners and crystallizers:

| Example 3 - Yellow to Blue-Green | W/W (%) | CAS No. | Function |
|---|---|---|---|
| Performathox 450 ethoxylate* | 10.0 | 251553-55-6 | Surfactant |
| Performathox 480 ethoxylate* | 20.0 | 251553-55-6 | Surfactant |
| Foral AX-E 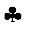 | 10.0 | 9005-00-9 | Tackifying Agent/Stabilizer |
| Ethylene Acrylic Acid AC-5120^{Ω} | 25.5 | 9010-77-9 & 79-10-7 | Stabilizer & Binding Agent |
| Unicid 350^{Ω} | 31.8 | 251554-90-2 &9002-88-4 | Crystallizer/Hardener/Stabilizer |
| Hostaphat™ CC-100^{>} | 0.2 | 3539-43-3 | Stabilizer |
| Irganox 1010 ^{□} | 2.0 | 1709-70-2 | Anti-Oxidant |
| Bromocresol Green Free Acid 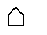 | 0.5 | 76-60-8 | Colorant |

| | | | |
|---|---|---|---|
| *Performathox 450 and Performathox 480 as supplied by Baker-Hughes of Houston, TX. 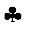 Foral AX-E as supplied by Eastman Chemicals of Kingsport, TN. ^{□}Irganox 1010 as supplied by BASF of Florham Park, NJ. ^{>}Hostaphat CC-100 as supplied by Clariant Inc. of Charlotte, NC. ^{Ω}Ethylene Acrylic Acid as supplied as AC-5120 by Honeywell Inc. of Morristown, NJ. ^{Ω}Unicid 550 as supplied by Baker-Hughes of Houston, TX. 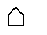 Bromophenol Green free acid as supplied by TCI Chemicals of Portland, OR. | | | |

Example 4 is another modification of Example 2 and Example 3 with the use of Unicid 425 along Unicid 550. Here, Example 4 is another wetness indicator composition that changes from a yellow dry state color to a bluish-green wet state color and it contains four acid stabilizers with Foral™ AX-E, Hostaphat™ CC-100, AC™-5120 from Honeywell Inc., and both Unicid™425 and Unicid™550. As noted, the acid stabilizers Unicid™ 425 and Unicid™550 also functions as crystallizers and hardeners. Thus, this Example 4 composition possesses both chemical stability due to the inclusion of acid stabilizers and physical stabilization due to the inclusion of crystallizers and hardeners which allow the WI composition to set up quickly into a hard solid on the substrate. Its quick solidification on the substrate during manufacturing prevents it from migrating into other regions of the diaper where pretriggerants might be present. Example 4's hardness as a solid also inhibits penetration of pretriggerants into the WI while the acid stabilizers maintain the protonated dry state colors of the colorants even in hot and humid environments. Here is the Example 4 composition which exemplifies not only chemical stabilization but also physical stabilization via the inclusion of hardeners and crystallizers:

| Example 4 - Yellow to Blue-Green | W/W (%) | CAS No. | Function |
|---|---|---|---|
| Performathox 450 ethoxylate* | 10.0 | 251553-55-6 | Surfactant |
| Performathox 480 ethoxylate* | 20.0 | 251553-55-6 | Surfactant |
| Foral AX-E 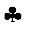 | 10.0 | 9005-00-9 | Tackifying Agent/Stabilizer |
| Ethylene Acrylic Acid AC-5120^{Ω} | 25.5 | 9010-77-9 & 79-10-7 | Stabilizer & Binding Agent |
| Unicid 425^{Ω} | 24.0 | 251554-90-2 &9002-88-4 | Crystallizer/Hardener/Stabilizer |
| Unicid 550^{Ω} | 7.3 | 251554-90-2 &9002-88-4 | Crystallizer/Hardener/Stabilizer |
| Hostaphat™ CC-100^{>} | 0.2 | 3539-43-3 | Stabilizer |
| Silicone Oil 10 cSt | 0.5 | 63148-62-9 | Plasticizer |
| Irganox 1010 ^{□} | 2.0 | 1709-70-2 | Anti-Oxidant |
| Bromocresol Green Free Acid 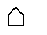 | 0.5 | 76-60-8 | Colorant |

| | | | |
|---|---|---|---|
| *Performathox 450 and Performathox 480 as supplied by Baker-Hughes of Houston, TX. 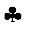 Foral AX-E as supplied by Eastman Chemicals of Kingsport, TN. ^{□}Irganox 1010 as supplied by BASF of Florham Park, NJ. ^{>}Hostaphat CC-100 as supplied by Clariant Inc. of Charlotte, NC. ^{Ω}Ethylene Acrylic Acid as supplied as AC-5120 by Honeywell Inc. of Morristown, NJ. ^{Ω}Unicid 550 as supplied by Baker-Hughes of Houston, TX. 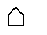 Bromophenol Green free acid as supplied by TCI Chemicals of Portland, OR. Silicone Oil at 10 cSt as supplied by Dow Corning, Midland, MI | | | |

Example 5 shows a wetness indicator composition with a yellow dry state that changes to purple upon contact with baby's urine. For this Example 5, there are multiple acidic stabilizers where the main stabilizer for the free acid of bromophenol blue is the ethylene acrylic acid copolymer. The free acid of cetyl phosphate from the Hostaphat CC-100 is a strong enough acid to protonate both the Phloxine B acid into its colorless form and the bromophenol blue into its acidic yellow form. The hydrogenated gum rosin trademarked as Foral AX-E from Eastman Chemicals can also function as both a tackifying agent along with functioning as an acid stabilizer. Being hydrogenated, the Foral AX-E is also of low color and low odor and is more stable than non-hydrogenated versions. The Hostaphat CC-100 cetyl phosphate stabilizer is acidic enough to protonate both the Phloxine B free acid and the free acid of bromophenol blue since the pKa of cetyl phosphate is lower than both of the colorants.

| Example 5 - Yellow to Purple | W/W (%) | CAS No. | Function |
|---|---|---|---|
| Performathox 450 ethoxylate * | 11.2 | 251553-55-6 | Surfactant |
| Performathox 480 ethoxylate * | 16.7 | 251553-55-6 | Surfactant |
| Foral AX-E 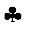 | 20.5 | 9005-00-9 | Tackifying Agent |
| Irganox 1010 ^{□} | 1.0 | 1709-70-2 | Anti-Oxidant |
| AC-5120 Ethylene Acrylic Acid^{Ω} | 45.0 | 9010-77-9 & 79-10-7 | Stabilizer & Binding Agent |
| Benzoflex 98-8^{‡} | 3.6 | 20109-39-1 | Plasticizer |
| Hostaphat CC-100^{>} | 0.8 | 3539-43-3 | Stabilizer |
| Tinuvin UV Light Protectants ^{∘} | 0.99 | 129757-67-1 & 127519-17-9 | UV Light Protectants |
| Bromophenol Blue Free Acid 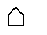 | 0.15 | 115-39-9 | Colorant |
| Phloxine B Acid 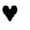 | 0.06 | 18472-87-2 | Colorant |

| | | | |
|---|---|---|---|
| *Performathox 420 and Performathox 480 as supplied by Baker-Hughes of Houston, TX. 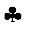 Foral AX-E as supplied by Eastman Chemicals of Kingsport, TN. ^{□}Irganox 1010 as supplied by BASF of Florham Park, NJ.. ^{Ω}Ethylene Acrylic Acid as supplied as AC-5120 by Honeywell Inc. of Morristown, NJ ^{‡}Benzoflex 98-8 as supplied by Eastman Chemicals of Kingsport, TN. ^{>}Hostaphat CC-100 as supplied by Clariant Inc. of Charlotte, NC ^{∘}Tinuvin UV light protectants are a 50%-50% blend of Tinuvin 123 and Tinuvin 384-2 as supplied by BASF of Florham Park, NJ.. 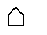 Bromophenol Blue free acid as supplied by TCI Chemicals of Portland, OR. 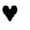 Phloxine B Acid as supplied by TCI Chemicals of Portland, OR. | | | |

Colorants that may be used in the present invention include, but are not limited to, the colorants listed in Table 1 below. Table 1 also indicates the low pH color, the pH transition range, high pH color, and pKa of each colorant. (Orndorff, W.R.;Purdy, A.C. J. Am. Chem. Soc. 1926, 48, 2216; also in the book "The Sigma Aldrich Handbook of Stains, Dyes, and Indicators," by Floyd J. Green, 2nd printing published in 1991 by the Aldrich Chemical Company of Milwaukee, WIS, ; See, also, "The Handbook of Acid-Base Indicators," by R.W. Sabnis and published in 2008 by CRC Press of NY, NY).

**Table 1:**

| COLORANT | CAS # | Low pH Color | pH Transition Range | High pH Color | pKa |
|---|---|---|---|---|---|
| Gentian Violet (Crystal Violet) | 548-62-9 | Yellow | 0.0 to 2.0 | Blue-Violet | 1.1 & 1.8 |
| Acid Phloxine B (free acid form; D&C Red 27) | 13473-26-2 | Colorless | 1.1 to 3.3 | Purple | 2.9 |
| Phloxine B (sodium salt; D&C Red 28) | 18472-87-2 | Colorless | 1.1 to 3.3 | Purple | 2.9 |
| Methyl Violet | 52080-58-7 | Yellow | 0.2 to 1.8 | Purple | 0.8 |
| Malachite Green (Acidic pH range) | 2437-29-8 | Yellow | 0.0 to 2.0 | Green | 1.3 |
| Malachite Green (Alkaline pH range) | 2437-29-8 | Blue-green | 11.6 to 14.0 | Colorless | 12.8 |
| Bromophenol Blue Free Acid | 115-39-9 | Yellow | 3.0 to 4.6 | Blue | 4.0 |
| Methyl Orange | 547-58-0 | Red | 3.2 to 4.4 | Yellow | 3.4 |
| Resazurin | 550-82-3 | Orange | 3.8 to 6.5 | Purple | 5.1 |
| Ethyl Red | 76058-33-8 | Red | 4.5 to 6.5 | Yellow | 5.4 |
| Bromocresol Green Free Acid | 76-60-8 | Yellow | 3.8 to 5.4 | Blue-Green | 4.8 |
| Quinaldine Red | 117-92-0 | Colorless | 1.4 to 3.2 | Red | 2.6 |
| Bromocresol Purple Free Acid | 115-40-2 | Yellow | 5.2 to 6.8 | Purple | 6.3 |
| Thymo lphthalein | 125-20-2 | Colorless | 9.3 to 10.5 | Blue | 9.8 |
| Acid Fuchsin | 3244-88-0 | Red | 12.0 to 14.0 | Colorless | 13 |
| Nile Blue | 2381-85-3 | Blue | 9.4 to 11.0 | Purple-Red | 9.7 |
| Aniline Blue (also known as Methyl Blue) | 28983-56-4 | Blue | 9.4 to 14.0 | Orange | 11.7 |
| Indigo Carmine | 860-22-0 | Blue | 11.5 to 14.0 | Yellow | 12.7 |

The wetness indicators of the present invention may comprise from about 0.01% to about 15.0% by weight of colorant(s). The stabilizer(s), when present, is /are typically employed in compositions at levels which are effective at stabilizing the colorant, from about 0.001% to about 30%, from about 0.1% to about 15%, and also from about 0.5 % to about 10%, by weight of the composition.

The wetness indicator may comprise additional colorant(s). Additional suitable fluid colorants include water soluble colorants like direct dyes, acid dyes, base dyes, and various solvent-soluble colorants. Examples of colorants further include, but are not limited to, organic dyes, inorganic pigments, colored macromolecules, colored nanoparticles and materials. In some embodiments, a permanent colorant may be added. Some examples of oil soluble permanent colorants include D&C Yellow No. 11, D&C Red No. 17, D&C Red No. 21, D&C Red No. 27, D&C Red No. 31, D&C Violet No. 2, D&C Green No. 6, FD&C Red 3, D&C Orange No. 4, D&C Orange No. 17, and D&C Orange No. 5. Additional permanent colorants include Pigment Red 146 (CAS#5280-68-2), Pigment Red 122 (CAS#980-26-7), Pigment Orange 16 (CAS#6505-28-8), red beet extract, Manganese Phthalocyanine and other metallized phthalocyanines like copper phthalocyanines and metallized and alkylated porphyrin or phthalocyanines, and beta-carotene and mixtures thereof. Further appropriate additional colorants may include those listed in U.S. Ser. No. 62/147,258.

Appropriate stabilizers include, but are not limited to, those listed in the following Table 2, along with their pKa value(s). Some embodiments may use two, three, four, or more stabilizers. As noted above, the function of acid stabilizers is to keep the pH indicator colorant in a protonated state below its pKa value in the dry wetness indicating state. Thus, since pH indicator colorants have a multitude of different pKa's, a variety of different acids with varying pKa values are required to stabilize these various pH indicator colorants although in certain instances, one very strong acid stabilizer may perform very well with a variety of colorants with pKa values below a value of 7. Alkaline stabilizers may also be required and here the function of the alkaline or basic stabilizer is to keep the pH indicator colorant in its conjugated basic form above its pKa value in the dry wetness indicating state. For the table of acid and alkaline stabilizers below, some of them have more than one pKa value because that particular molecule has more than one acid or alkaline moiety. For example, citric acid possesses three acidic protons with each having different acid strengths. Most frequently, the first pKa is the lowest since the first proton is most frequently the most acidic. Upon release of the first proton, the molecule becomes anionic and that negative charge makes it more difficult for the citric acid molecule to release the second proton. Thus, the second proton is less acidic than the release of the first proton and the second pKa (2) is higher than the first pKa (1). Finally, upon release of two protons from citric acid, the molecule now possesses a negative II charge and this attracts the last remaining positively charged proton such that it is the weakest proton of the three on the citric acid molecule. Thus, citric acid's pKa (3) is larger than its pKa (2) for its second of three protons which is larger than the most acidic pKa (1) proton. In addition, some acid and alkaline stabilizers may be complex mixtures containing molecules with various pKa values. For example and as noted, the cetyl phosphate acid stabilizer as sold as Hostaphat™ CC-100 from Clariant Inc. can contain traces of phosphoric acid and other acidic components. The key is that the acid or basic stabilizer has one or more components that can stabilize the colorant in its dry state. For acid stabilizers, it must be more acidic and possess a lower pKa than the colorant it must acidify. For basic stabilizers, it must be more alkaline and possess a higher pKa than the colorant so the alkaline colorant is maintained in its basic form in the dry state of the wetness indicator composition.

**Table 2:**

| STABILIZER NAME | | pKa (1) | pKa (2) | pKa (3) | pKa (4) | pKa (5) |
|---|---|---|---|---|---|---|
| Acetamide | | 0.6 | | | | |
| Acetic acid | | 4.8 | | | | |
| Acetoacetic acid | | 3.6 | | | | |
| Adipic acid | | 4.4 | 5.4 | | | |
| Alkyl Sulfonic Acids | | ∼1 | | | | |
| 2-Aminobenzoic acid | | 2.1 | 4.9 | | | |
| Ammonia | | 9.2 | | | | |
| Aniline | | 4.6 | | | | |
| Arginine | | 1.8 | 9.0 | 12.5 | | |
| Ascorbic acid | | 4.1 | 11.8 | | | |
| Aspartic acid | | 2.0 | 3.9 | 10.0 | | |
| Barbituric acid | | 4.0 | | | | |
| Benzenesulfonic acid | | 0.7 | | | | |
| Benzoic acid | | 4.2 | | | | |
| Benzylamine | | 9.3 | | | | |
| Betaine | | 1.83 | | | | |
| Boric acid | | 9.3 | 12.7 | 13.8 | | |
| Butanoic acid | | 4.8 | | | | |
| Butylamine | | 10.8 | | | | |
| Carbonic acid | | 6.4 | 10.3 | | | |
| Catechol | | 9.4 | 12.8 | | | |
| Cetyl Phosphate | | ∼2 | | | | |
| Chloroacetic acid | | 2.9 | | | | |
| Citric acid | | 3.1 | 4.8 | 6.4 | | |
| m-Cresol | | 10.0 | | | | |
| Cysteine | | 1.7 | 8.4 | 10.8 | | |
| Decylamine | | 10.6 | | | | |
| Dichloroacetic acid | | 1.3 | | | | |
| Diethylamine | | 10.9 | | | | |
| Diisopropylamine | | 11.0 | | | | |
| Dimethylamine | | 10.8 | | | | |
| Dimethylglyoxime | | 10.7 | 12.0 | | | |
| Dinicotinic acid | | 2.8 | | | | |
| Ethanolamine | | 9.5 | | | | |
| Ethylamine | | 10.6 | | | | |
| Ethylenediamine | | 6.8 | 9.9 | | | |
| Ethylenediaminetetraace tic acid (EDTA) | | -0.21 | 1.5 | 2.2 | 3.1 | 6.7 |
| Ethyleneimine | | 8.0 | | | | |
| Formic acid | | 3.7 | | | | |
| Fumaric acid | | 3.1 | 4.5 | | | |
| L-Glutamic acid | | 2.2 | 4.4 | 9.9 | | |
| L-Glutamine | | 2.2 | 9.1 | | | |
| L-Glutathione | | 2.12 | 3.59 | 8.75 | 9.65 | |
| Glyceric acid | | 3.5 | | | | |
| Glycine | | 2.3 | 9.8 | | | |
| Glycolic acid | | 3.8 | | | | |
| Glyoxylic acid | | 3.2 | | | | |
| Heptanedioic acid | | 4.7 | | | | |
| Heptanoic acid | | 4.9 | | | | |
| Heptylamine | | 10.7 | | | | |
| Hexamethylenediamine | | 11.9 | 10.8 | | | |
| Hexanoic acid | | 4.8 | | | | |
| Hexylamine | | 10.6 | | | | |
| Hydrogen chloride | | -7 | | | | |
| Hydroquinone | | 10.3 | | | | |
| Hydroxylamine | | 5.9 | | | | |
| Lactic acid | | 3.9 | | | | |
| Maleic acid | | 1.9 | 6.3 | | | |
| Malic acid | | 3.5 | 5.1 | | | |
| Malonic acid | | 2.847 | 5.696 | | | |
| 4-Methylpentanoic acid | | 4.8 | | | | |
| Nicotine | | 3.1 | 8.0 | | | |
| Nitrous acid | | 3.1 | | | | |
| Octadecylamine | | 10.6 | | | | |
| Octanedioic acid | | 4.5 | | | | |
| Octanoic acid | | 4.9 | | | | |
| Oxalic acid | | 1.2 | 4.3 | | | |
| Pentanoic acid | | 4.8 | | | | |
| Perchloric acid | | -10 | | | | |
| p-Periodic acid | | 1.5 | 8.3 | | | |
| 1,10-Phenanthroline | | 4.8 | | | | |
| Phenol | | 10.0 | | | | |
| Phenylacetic acid | | 4.3 | | | | |
| Phenylalanine | | 2.2 | 9.3 | | | |
| Phenylethylamine | | 9.8 | | | | |
| Phenylglycine | | 1.8 | 4.4 | | | |
| Phosphoric acid | | 2.1 | 7.2 | 12.4 | | |
| m-Phthalic acid | | 3.5 | 4.6 | | | |
| o-Phthalic acid | | 2.9 | 5.4 | | | |
| p-Phthalic acid | | 3.5 | 4.8 | | | |
| Picolinic acid | | 1.1 | 5.2 | | | |
| Picric acid | | 0.4 | | | | |
| Propanoic acid | | 4.9 | | | | |
| Propylamine | | 10.6 | | | | |
| 3-Pyridinecarboxylic acid | | 4.9 | | | | |
| 4-Pyridinecarboxylic acid | | 5.0 | | | | |
| Pyrimidine | | 6.3 | | | | |
| Pyrocatechol | | 9.4 | 12.8 | | | |
| Pyrophosphoric Acid | | 1.5 | 2.4 | 6.6 | 9.2 | |
| Pyrrolidine | | 11.3 | | | | |
| Pyruvic acid | | 2.4 | | | | |
| Quinine | | 4.1 | 8.5 | | | |
| Quino line | | 4.9 | | | | |
| Resorcinol | | 9.3 | 11.1 | | | |
| Salicylic acid | | 3.0 | 13.7 | | | |
| Selenic acid | | 1.9 | | | | |
| Selenous acid | | 2.6 | 8.3 | | | |
| Serine | | 2.2 | 9.0 | | | |
| o-Silicic acid | | 9.7 | 11.7 | | | |
| m-Silicic acid | | 9.7 | 12 | | | |
| Succinic acid | | 4.2 | 5.6 | | | |
| Sulfuric acid | | -3 | 2.0 | | | |
| Sulfurous acid | | 1.9 | 7.2 | | | |
| d-Tartaric acid | | 3.0 | 4.4 | | | |
| meso-Tartaric acid | | 3.2 | 4.8 | | | |
| Terephthalic acid | | 3.5 | | | | |
| m-Toluic acid | | 4.3 | | | | |
| o-Toluic acid | | 3.9 | | | | |
| p-Toluic acid | | 4.4 | | | | |
| Trichloroacetic acid | | 0.9 | | | | |
| Triethano lamine | | 7.8 | | | | |
| Triethylamine | | 10.7 | | | | |
| Trimethylacetic acid | | 5.0 | | | | |
| Trimethylamine | | 9.8 | | | | |
| Tris(hydroxymethyl)-aminomethane (tris) | | 8.1 | | | | |
| Tyramine | | 9.8 | 10.5 | | | |
| Tyrosine | | 2.2 | 9.2 | 10.5 | | |
| Uric acid | | 3.9 | | | | |

Table 3 below shows the hot to cold solidification rate, set point temperature, cold to hot melting rate, melt point temperature, and infinite shear rate for seven wetness indicators. WI Reference A and Reference B are comparative wetness indicators from commercially available products. WI Examples 1-5 correspond with the formula examples given above.

In general, as discussed above, to exhibit improved stability a wetness indicator must have a least three of the five parameters as follows:
(i) a hot to cold solidification rate of Delta(G')/Delta(°C) from about 3,800 to about 27,000 Pa/°C ;
(ii) a set point temperature from about 60°C to about 110°C;
(iii) an infinite shear rate from about 0.02 to about 0.5 sec⁻¹;
(iv) a cold to hot melting rate from about 3,700 to about 11,000 Pa/°C ; and
(v) a melt point temperature from about 58°C to about 135 °C.

The data in Table 3 shows that the inventive formulas have at least three of the parameters and thus can exhibit improved stability. In some embodiments, the inventive wetness indicator may have three of the five parameters described above, in some embodiments, four of the five parameters, and in some embodiments five of the five parameters.

**Table 3**

| | Hot to Cold Solidification Rate | | Cold to Hot Melting Rate | | Infinite Shear Rate (sec⁻¹) | |
|---|---|---|---|---|---|---|
| Sample | Delta G/C: | Set point C | Delta G/C | Melt point C | | Met Critieria? |
| WI Reference A | 9893.7 | 22.15 | 6648.6 | 30.7 | 3.32 | No |
| WI Reference B | 29373.6 | 48.28 | 14244 | 70.6 | 1.54 | No |
| WI Formula Example 1 | 22618.86 | 89.5 | 8941.81 | 113.82 | 0.027 | Yes |
| WI Formula Example 2 | 17819.8 | 90.54 | 6088.99 | 102.75 | 0.07 | Yes |
| WI Formula Example 3 | 5779 | 80.5 | 4978.1 | 81.7 | 0.076 | Yes |
| WI Formula Example 4 | 13564.06 | 89.78 | 8060.8 | 100.34 | 0.107 | Yes |
| WI Formula Example 5 | 4817.94 | 79.45 | 4704.04 | 73.5 | 0.301 | Yes |

Table 4 shows the needle penetration at two temperatures for Examples 1-5 of the inventive wetness indicators. The low needle penetrations (measured according to ASTM D1321-04 in dmm or decimillimeters) indicate the high level of hardness for the wetness indicators, which can lead to improved stability.

**Table 4**

| | Needle Penetration at 23°C (dmm) | Needle Penetration at 55°C (dmm) |
|---|---|---|
| WI Formula Example 1 | 0 | 32 |
| WI Formula Example 2 | 2 | 43 |
| WI Formula Example 3 | 5 | 102 |
| WI Formula Example 4 | 2 | 66 |
| WI Formula Example 5 | 7 | 115 |

### Hot Melt Binding Matrix

The wetness indicating compositions that are utilized in this invention comprise a hot melt binding matrix. Processing a hot melt binding matrix involves melting the components together at an elevated temperature, typically from at least about 50°C to about 170°C, in some embodiments, from about 60°C to about 130°C, in some embodiments from about 80°C to about 120°C. In order to be hot melt processable, the wetness indicator composition must be heated to a temperature high enough so as to insure the adhesive flows readily but not so hot so as to cause degradation at an unacceptable rate. Thus, it is common to add an anti-oxidant to hot melt compositions in order to slow down the decomposition rate. It may be difficult to achieve compatibility and stability of such wetness indicating components if processed at room temperature. It may also be difficult under some printing processes to print such compositions onto a substrate. But the present invention's components are melted together at elevated temperatures, and the hot melt liquid is applied and adhered to a substrate while at an elevated temperature to keep the composition in its liquid molten state.

The hot melt binding matrix may comprise binding agents that can be any material that immobilizes the colorant, or combination of colorants, within the matrix to hinder leaching of the colorant(s) into a diaper core or other regions of an absorbent article. To optimize the contrast and vibrancy of the colors, it is much preferred to "lock" the colorant within the matrix before and after contact with a fluid like urine. The binding agents can not only hinder the leaching of the color outside of the matrix, but also aid in binding the entire wetness indicator composition to a component of the absorbent article. For example, the binder can aid in forming a strong bond between the surface of the diaper backsheet and the wetness indicator composition.

There are various materials which may be suitable for use as a binding agent in a hot melt binding matrix for the wetness indicators of the present invention. A number of different polymers and blends of polymers used in hot melt adhesives may be used as the primary binding agent to combine and mix the pH indicating colorants with the acid or alkaline stabilizer and other optional ingredients such as tackifiers, waxes, surfactants, viscosity modifiers, fillers, anti-oxidants, UV stabilizers and other colorants. Some of these materials can also function as crystallizers or hardeners to contribute to the stability of the wetness indicator composition.

Such hot melt polymers, copolymers, terpolymers, and other materials that can function as a binding agent include ethylene vinyl acetates (EVA), polyolefins like low density polyethylene (LDPE) and high density polyethylene (HDPE), atactic polypropylene and polypropylene homopolymers, propylene-ethylene copolymer waxes like Clariant's Licocene PP-1502, oxidized polyethylene like Honeywell's A-C 6702 and A-C 330 and Henkel's Technomelt (REGISTERED™ symbol) line of polyolefins. Polyamides like Henkel's Macromelt (REGISTERED™ symbol here) 6072. Other hot melt components that can function as a binding agent include polymethyl methacrylate, ethylene vinyl acetates (EVA) like Dupont Elvax (trademark symbol) line of EVA's, polymethacrylic acid, polyacrylic acid, ethylene-acrylic acid copolymers (EAA) like Honeywell's A-C 5120, fully and partially neutralized salts of the ethylene-acrylic acid copolymers, ethylene-ethyl acetate, polyacrylates, oxidized ethylene-vinyl acetate copolymers like Honeywell's A-C 645P, ethylene maleic anhydride copolymers, propylene maleic anhydride copolymers, polyethylene imines (PEI) like BASF's Lupasol (registered trademark symbol), polyurethanes like the polycaprolactone thermoplastic polyurethane named Pearlbond™ 120 from Lubrizol Inc., polyacryl amides, branched copolymers comprising monomeric units derived from acrylic acid and/or quaternary ammonium compounds and/or acrylamide, branched copolymers comprising one or more monomeric units derived from quaternary ammonium compounds, amine compounds, acrylamide compounds, acrylic acid compounds and mixtures thereof at various weight ratios within the polymer. Another example is a copolymer of acrylamide reacted with one or more other nonionic monomers, for example non-acrylamide monomers, such as hydroxyalkylacrylate, for example hydroxypropylacrylate. Another example is a branched copolymer of acrylamide reacted with bismethyleneacrylamide, a crosslinking agent, that converts a typical linear polyacrylamide into a branched polymeric structure. Another copolymer example includes the reaction between a nonionic monomeric unit derived from an acrylamide compound and an anionic monomeric unit derived from acrylic acid or other suitable monomers that could become anionic where examples include anionic monomers selected from the group consisting of: monomers having at least one carboxylic function, for instance α,β-ethylenically unsaturated carboxylic acids or the corresponding anhydrides, such as acrylic, methacrylic or maleic acids or anhydrides, fumaric acid, itaconic acid, N-methacroylalanine, N-acryloylglycine, and their water-soluble salts, monomers that are precursors of carboxylate functions, such as tert-butyl acrylate, which, after polymerization, give rise to carboxylic functions by hydrolysis, monomers having at least one sulfate or sulfonate function, such as 2-sulfooxyethyl methacrylate, vinylbenzene sulfonic acid, allyl sulfonic acid, 2-acrylamido-2-methylpropane sulfonic acid (AMPS), sulfoethyl acrylate or methacrylate, sulfopropyl acrylate or methacrylate, and their water-soluble salts, monomers having at least one phosphonate or phosphate function, such as vinylphosphonic acid, etc., the esters of ethylenically unsaturated phosphates, such as the phosphates derived from hydroxyethyl methacrylate (Empicryl 6835 from Rhodia) and those derived from polyoxyalkylene methacrylates, and their water-soluble salts, and 2-carboxyethyl acrylate (CEA). Not to be bound by theory, but the inclusion of potential anionic moieties within the polymer backbone can aid in decreasing the leaching of a blue cationic form of a colorant by forming complex between the polymeric anionic and cationic colorant. Other binding agents can form strong associations with colorant molecules through other bonding forces that include van der Waals and hydrogen bonding.

The hot melt may also comprise polymers with a cationic monomeric unit, such as a cationic monomeric unit derived from cationic monomers selected from the group consisting of: N,N-(dialkylamino-ω-alkyl)amides of α,β-monoethylenically unsaturated carboxylic acids, such as N,N-dimethylaminomethylacrylamide or -methacrylamide, 2-(N,N-dimethylamino)ethylacrylamide or-methacrylamide, 3-(N,N-dimethylamino)propylacrylamide or -methacrylamide, and 4-(N,N-dimethylamino)butylacrylamide or -methacrylamide, α,β-monoethylenically unsaturated amino esters such as 2-(dimethylamino)ethyl acrylate (DMAA), 2-(dimethylamino)ethyl methacrylate (DMAM), 3-(dimethylamino)propyl methacrylate, 2-(tert-butylamino)ethyl methacrylate, 2-(dipentylamino)ethyl methacrylate, and 2-(diethylamino)ethyl methacrylate, vinylpyridines, vinylamine, vinylimidazolines, monomers that are precursors of amine functions such as N-vinylformamide, N-vinylacetamide, which give rise to primary amine functions by simple acid or base hydrolysis, acryloyl- or acryloyloxyammonium monomers such as trimethylammonium propyl methacrylate chloride, trimethylammonium ethylacrylamide or -methacrylamide chloride or bromide, trimethylammonium butylacrylamide or -methacrylamide methyl sulfate, trimethylammonium propylmethacrylamide methyl sulfate, (3-methacrylamidopropyl)trimethylammonium chloride (MAPTAC), (3-methacrylamidopropyl)trimethylammonium methyl sulphate (MAPTA-MES), (3-acrylamidopropyl)trimethylammonium chloride (APTAC), methacryloyloxyethyl-trimethylammonium chloride or methyl sulfate, and acryloyloxyethyltrimethylammonium chloride; 1-ethyl-2-vinylpyridinium or 1-ethyl-4-vinylpyridinium bromide, chloride or methyl sulfate; N,N-dialkyldiallylamine monomers such as N,N-dimethyldiallylammonium chloride (DADMAC); polyquaternary monomers such as dimethylaminopropylmethacrylamide chloride and N-(3-chloro-2-hydroxypropyl)trimethylammonium (DIQUAT) and 2-hydroxy-N¹-(3-(2((3-methacrylamidopropyl)dimethylamino)-acetamido)propyl)-N¹,N¹,N³,N³,N³-pentamethylpropane-1,3-diaminium chloride (TRIQUAT). In one example, the cationic monomeric unit comprises a quaternary ammonium monomeric unit, for example a monoquaternary ammonium monomeric unit, a diquaternary ammonium monomeric unit and a triquaternary monomeric unit. In one example, the cationic monomeric unit is derived from MAPTAC. In another example, the cationic monomeric unit is derived from DADMAC. In still another example, the cationic monomeric unit is derived from 2-hydroxy-N¹-(3-(2((3-methacrylamidopropyl)dimethylamino)-acetamido)propyl)-N¹,N¹,N³,N³,N³-pentamethylpropane-1,3-diaminium chloride. Other polymers that can make up the hot melt include polyamines, polypryrroles, polyimidazoles, polycarbonates, polyesters, styrene block copolymers, PVP, PVP/VA copolymer like Ashland Chemical's S-630 PVP/VA, polyacrylamide, polyacryldextran, polyalkyl cyanoacrylate, cellulose acetate, cellulose acetate butyrate, cellulose nitrate, methyl cellulose and other cellulose derivatives, chitosan and chitosan derivatives, chitin and chitin derivatives, nylon 6,10, nylon 6,6, nylon 6, polyterephthalamide and other polyamides, polycaprolactones, polydimethylsiloxanes and other siloxanes, silicone rubbers, aliphatic and aromatic polyesters, polyethylene oxide, polyglycolic acid, polylactic acid and copolymers, poly(methyl vinyl ether/maleic anhydride), polystyrene, polyvinyl acetate phthalate, polyvinyl alcohol and its copolymers, polyvinylpyrrolidone, shellac, starch and modified starches, fatty alcohols, primary alcohols of long carbon chain lengths of C24 to C50, ethoxylated fatty alcohols, ethoxylated primary alcohols of chain lengths of C24 to C50, fatty acids, and waxes such as paraffinic and microcrystalline, synthetic waxes like polyethylene waxes, natural waxes like beeswax, carnauba wax and mixtures thereof. As noted previously, some of the these waxes and higher molecular weight materials can function also as both hardeners and crystallizers.

In some embodiments, the binding agent may be a hot melt adhesive, in some embodiments, a solvent-based binding matrix. Additional components of a hot melt adhesive binding matrix may include base polymers, tackifiers, waxes, rubbers, wetting agents, and/or anti-oxidants. Examples of base polymers used in hot melt adhesives may include ethylene-vinyl acetate (EVA) copolymers like those of the Elvax brand name and marketed by DuPont Incorporated; styrenic block copolymers like those from Kraton Incorporated, ethylene/acrylic acid copolymers like the AC brand marketed by Honeywell Incorporated, vinyl pyrrolidone/vinyl acetate copolymers, vinyl pyrrolidone homopolymers like those marketed by BASF Incorporated under the trade name of Luviskol, polyamides; , ethylene-acrylate copolymers; ethylene-vinylacetate-maleic anhydride terpolymers; ethylene-acrylate-maleic anhydride terpolymers; polyolefins such as low density and high density polyethylene, polypropylene, oxidized polyethylene, polybutene-1; amorphous polyolefins like amorphous atactic polypropylene (APP), amorphous poly-propylene/ethylene (APE), amorphous poly-propylene/butene (APB), amorphous poly-propylene/hexene (APH), and amorphous polypropylene/ethylene/butene ; polyamides; styrene/acrylic polymers and modified styrene/acrylic polymers; polycarbonates; silicone rubbers; polypyrrole based polymers; thermoplastic elastomers like natural and synthetic polyisoprene, polybutadiene rubber, butyl rubber, chloroprene rubber, ethylene-propylene rubber, epichlorohydrin rubber, polyacrylic rubber, polyether block amides; alkyd resins, amino resins, , epoxy resins, fluoropolymers. The binding agent may be employed in compositions at levels which are effective at immobilizing and stabilizing the colorant in its first state, including from about 1% to about 90%, from about 10% to about 75%, and from about 15% to about 65%, by weight of the wetness indicator composition.

The binding matrix may comprise a first and second binding agent. The second binding agent may be any material which may immobilize the colorant when the colorant is in its final color state. This immobilization helps to bind the colorant within the wetness indicator composition to prevent it from leaching to other regions of the diaper such as the diaper core. It should be noted that similar to the first binding agent, the second binding agent can function not only to hinder the leaching of the colorant outside of the wetness indicator composition but can also aid in bonding the entire wetness indicator composition to the material of interest within the absorbent article. For example, the second binding agent may aid in bonding the wetness indicator composition to the backsheet of the diaper. There are various materials which may be suitable for use as an additional binding agent for the wetness indicators of the present invention. For example, the binding agent might be a cationic agent to complex with anionic colorants. Or, the binding agent could be an anionic agent to complex with a cationic colorant like the blue and ring opened form of crystal violet lactone. In one embodiment, a binding agent may be selected from, but are not limited to, the second binding agents disclosed in USPN 6,904,865 to Klofta.

Tackifiers suitable for hot melt adhesives include, without being limited to, natural resins like the copal type, the damar type, the mastic type, the sandarac type, and mixtures thereof, ; rosins, their esters and their modified derivatives, both fully and partially hydrogenated, like modified tall oil rosins with Sylvaros PR-R™ from Arizona Chemical™ being an example; polymerized rosins like Sylvaros PR 295™ from Arizona Chemical™, partially dimerized gum rosins like Eastman™ Chemical Inc.'s Poly-Pale™, terpenes and modified terpenes; aliphatic, cycloaliphatic, and aromatic resins like C5 aliphatic resins, C9 aromatic resins, and C5/C9 aromatic/aliphatic resins, acidic rosins and acidic hydrogenated resins like Pinova's Foral AX synthetic resin, Eastman Chemical's fully hydrogenated rosin like its Foral AX-E, alkyl resins, phenolic resins and terpene-phenolic resins like Sylvares™ TP-2040 from Arizona Chemical Inc., hydrogenated hydrocarbon resins and their mixtures.

Tackifiers may be employed in the wetness indicator compositions at levels from about zero to about 60% or from about zero to about 40%, by weight of the composition.

Waxes suitable for hot melt adhesives include, without being limited to, synthetic waxes like paraffin and microcrystalline waxes; polyethylene waxes; polyethylene glycol type waxes like those trademarked as the Carbowax brand; oxidized polyethylene waxes; polymethylene waxes, the bis-stearamides like N,N'-ethylene bis-stearamide trademarked as Acrawax from Lonza Incorporated, highly branched polymer waxes like Vybar™ from Baker Hughes; fatty amide waxes; waxes that are copolymers of ethylene or propylene with maleic anhydride and/or maleic esters; natural and synthetic waxes like beeswax, soywax, carnuba, ozokerite, ceresin, montan wax; waxes derived from both the Fisher-Tropsch and Ziegler-Natta processes; water soluble waxes, polyalkylene wax, and silicone waxes. Many of these waxes are especially suitable for hot melt type wetness indicators due to their ability to function as crystallization agents and hardeners.

Waxes may be employed in the wetness indicator compositions at levels from about zero to about 80% or from about zero to about 70%, by weight of the composition.

Additional additives for hot melt adhesives may include plasticizers, like glyceryl tribenzoate, benzoate esters like Eastman™ Chemicals Benzoflex™ 9-88, alkyl benzoates, C12-15 alkyl benzoate like Akzo's Dermol 25B, C2-C22 alkyl benzoates where the alkyl group is straight or branched or mixtures thereof, alkyl citrates, phthalate esters, paraffin oils, silicone oils, and polyisobutylene; UV stabilizers; biocides and antimicrobial preservatives; antioxidants, like BHT, phospites and phosphates; antistatic agents; pigment, particle and powder wetting agents like polyhydroxystearic acid, polyglyceryl-4 isostearate, hexyl laurate, esters like isopropyl myristate, propylene carbonate, isononyl isononanoate, glyceryl behenate/eicosadioate, trihydroxystearin, C12-15 alkyl benzoate, , castor oil; and viscosity modifiers. The wetting agent can be a combination of an ester like isononyl isononanoate and a surfactant like polyhydroxystearic acid. Optionally, solvents like mineral oil, isoparaffins, alkanes, silicone fluids, esters, alcohols, polyethylene glycols, glycerin, glycols, can be added to reduce the viscosity of the composition or to increase the solubility of other ingredients or change other strategic properties of the wetness indicator composition.

The hotmelt matrix may contain also mineral fillers, provided that they do not interfere with the color change of the pH indicators contained in the composition of the present invention. For example an acidic filler like precipitated silica may function both as an effective hardener of the formulation while keeping the desired acidic environment.

The matrix, including both the first and second binding agents, may be employed in wetness indicator compositions at levels which are effective at immobilizing and stabilizing the colorant, including from about 5% to about 95%, from about 10% to about 80%, and from about 25% to about 75%, by weight of the wetness indicator composition.

### Additional Ingredients

Additional ingredients may include, for example, at least a surfactant, a structural adjunct, and/or solvents. When present, such ingredients are typically employed in the composition at levels that are effective at providing the benefits of the ingredient or ingredients, such as, for example, from about 0.001% to about 50%, from about 0.1% to about 40%, or from about 1% to about 35%, by weight of the composition. Solvents may include a liquid, gel or semi-solid material. The solvent may be a thixotropic material, paste, an alcohol, ethylene glycol monobutyl ether, mineral oil, esters, silicone fluids and modified silicone fluids, isoparaffins, alkanes, low molecular weight polyethylene glycols like PEG-200, glycerin, glycols, a non-flammable solvent, an adhesive material, or other organic species. Preferred solvents may comprise alcohols, acetates, and combinations thereof.

Other suitable solvents that may be effective cetyl alcohol (fatty alcohol), dimethicone silicone, isopropyl lanolate, myristate, palmitate, lanolin, lanolin alcohols and oils, octyl dodecanol, oleic acid (olive oil), panthenol (vitamin B-complex derivative), stearic acid and stearyl alcohol, butylene glycol and propylene glycol, cyclomethicone (volatile silicone), glycerin, aloe, petrolatum, and so forth. Adhesives that may be useful include, for example, those based on alkyds, animal glues, casein glues, cellulose acetates, cellulose acetate butyrates, cellulose nitrates, ethyl celluloses, methyl celluloses, carboxy methyl celluloses, epoxy resins, furan resins, melamine resins, phenolic resins, unsaturated polyesters, polyethylacrylates, poly- methylmethacrylates, polystyrenes, polyvinylacetates, polyvinylalcohols, polyvinyl acetyls, polyvinyl chlorides, polyvinyl acetate chlorides, polyvinylidene copolymers, silicones, starched based vegetable glues, polyurethanes, acrylonitrile rubbers, polybutene rubbers, chlorinated rubbers, styrene rubbers, and so forth. Waxes such as, for example, polyolefin waxes, bees waxes, and so forth, and gels such as, for example, glycol dimethacrylate, chitosan, polyacrylates, hydroxypropylcellulose, gelatin, and so forth, may also be useful to effect the color change.

Surfactants that are suitable for the present invention may include, for example, ethoxylated alcohols, fatty alcohols, high molecular weight alcohols, sorbitan esters, ethoxylated sorbitan esters like Tween™ 40 from Croda, the ethoxylated pareth surfactants like Performathox™ 420 and Performathox ™ 450 and Performathox™ 480 and mixtures thereof from Baker Hughes Inc. Inc., ethoxylated esters, glycerol based esters, derivatized polymers; anionic and cationic and amphoteric surfactants, alkoxylated alkylates such as PEG-20 stearate, ethoxylated alcohols like the BRIJ™ materials from Croda Incorporated where Brij™ S-20/Steareth-20 and Brij™L-23 and Brij™S2/Steareth-2 are examples, end group-capped alkoxylated alcohols, alkoxylated glyceryl and polyglyceryl alkylates such as PEG-30 glyceryl stearate, glyceryl alkylates such as glyceryl stearate, low HLB emulsifiers like sorbitan esters where Span™60 from Croda Inc. is an example, alkoxylated hydrogenated castor oil, alkoxylated lanolin and hydrogenated lanolin, alkoxylated sorbitan alkylates, sugar derived surfactants such as the alkyl glycosides and sugar esters, poloxamers, polysorbates, and sulfo succinic acid alkyl esters like Aerosol™ OT-SE from Cytec is an example. Further examples include nonionic surfactants and amphoteric surfactants and any combination thereof; specificallydiethylhexylsodiumsulfosuccinate, available as MONOWET MOE75 from Croda, the sodium dioctyl sulfosuccinate line of surfactants like Aerosol™ OT-100 from Cytec Inc., the phosphate ester surfactants like Croda's Cetyl Phosphate tradenamed as Crodafos MCA or Croda's potassium salt form of Cetyl Phosphate tradenamed as Arlatone MAP160K, or Clariant's Cetyl Phosphate tradenamed as Hostaphat™ CC-100 and mixtures thereof, the alkyl benzene sulfonic acid and alkyl sulfonic acid surfactants and their corresponding salts like dodecylbenzene sulfonic acid tradenamed by AkzoNobel as Witconic 1298 Soft Acid or the counterpart with branching in the alkyl chain and tradenamed by AkzoNobel as Witconic 1298 Hard Acid, and mixtures thereof. Another example is 4-1-aminoethylphenolpolyoxyethylene fatty ethers, polyoxyethylene sorbitan esters, and polyoxyethylene fatty acid esters.

Other suitable surfactants may be neutral block copolymer surfactants, which can be selected from polyoxypropylene-polyoxyethylene block copolymer, poly [poly(ethylene oxide)-block-poly(propylene oxide)]copolymer or propylene glycol-ethylene glycol block copolymer. Suitable neutral polymeric surfactants include TWEEN surfactants, such as TWEEN 20 surfactant, TWEEN 40 surfactant and TWEEN 80 surfactant, and TRITON X-100 surfactant, which are available from Sigma-Aldrich, Incorporated. Other suitable neutral surfactants include polyethylene lauryl ether, polyoxyethylene nonyl phenyl ether, polyoxyethylene oleyl phenyl ether, polyoxyethylene sorbitan monolaurate, polyethylene glycol monostearate, polyethylene glycol sorbitan monolaurate, polyoxyethylenesorbitan monopalmitate, polyoxyethylenesorbitan monostearate, polyoxyethylenesorbitan monooleate, polyoxyethylenesorbitan trioleate, polypropylene glycol sorbitan monolaurate, polyoxypropylenesorbitan monopalmitate, polyoxypropylenesorbitan monostearate, polyoxypropylenesorbitan monooleate, polyoxypropylenesorbitan trioleate, polyalkyne glycol sorbitan monolaurate, polyalkyne glycol sorbitan monopalmitate, polyalkyne glycol sorbitan monostearate, polyalkyne glycol sorbitan monooleate, polyalkyne glycol sorbitan trioleate and mixtures of such neutral surfactants.

The neutral block copolymer based surfactants include PLURONIC series block copolymers, such as PLURONIC P84 or PLURONIC P85 surfactants, which are available from BASF Corporation.

Other suitable neutral block copolymer based surfactants include nonylphenol ethoxylates, linear alkyl alcohol ethoxylate, ethylene oxide-propylene oxide block copolymer, polyoxypropylene-polyoxyethylene block copolymer, polyalkylene oxide block copolymer and propylene glycol-ethylene glycol block copolymer.

It may be desirable to include additional stabilizer(s) when the colorant is a pH indicator and when the absorbent article could be stored under conditions of high humidity and high temperature or ultra intense UV light conditions. The inclusion of a stabilizer and UV light absorber or both is also especially important for new diaper designs where materials and/or chemicals are present that could potentially prematurely activate the color change of the colorant within the formulation. Also, the wetness indicator composition may be heated and mixed for long times and at high temperatures where the inclusion of anti-oxidants can slow down the degradation process. Thus, anti-oxidants like Irganox™ 1010 from BASF Inc. or Alvinox 100 from 3V-Sigma Inc. can aid in preventing premature oxidation and degradation of ingredients within the wetness indicating composition. In addition, if the wetness indicator composition might be exposed to ultraviolet light or intense sunlight for long periods of time, a UV stabilizer like Uvasorb™ S130 from 3V-Sigma or Escalol 577 (benzophenone-4, CAS #6628-37-1) from Ashland Chemicals might be added to inhibit photo-bleaching of the wetness indicator composition. Other effective UV stabilizers from BASF include Tinuvin-928 and Tinuvin-770 and Tinuvin-(384-2) and Tinuvin-123 and mixtures thereof.

Desiccants can stabilize the composition by trapping free water that could prematurely activate the wetness indicator composition. Examples of suitable desiccants include silica gel, bentonite clays, activated alumina, anhydrous calcium sulfate, copper(II) sulfate, and magnesium sulfate.

The present invention may include structural adjuncts, such as HLB (hydrophilic lipophilic balance) modifiers, viscosity modifiers, hardeners, wetting agents, anti-leaching aids, and/or colorant solubilizers. Suitable ones may include polymeric thickeners such as block copolymers having polystyrene blocks on both ends of a rubber chain, the aforementioned copolymers of ethylene and vinyl acetate (EVA), hydrogenated castor oil, other polymers, metals salts of fatty acids, silicas and or derivatized silicas, organoclays such as modified and unmodified hectorites and bentonites, modified clays such as modified laponite clays, dibenylidene sorbitol, alkyl galactomannan, aluminium magnesium hydroxide stearate/oil blends and lauroyl glutamic dibutylamide. Hardeners may include the aforementioned waxes, C14-22 fatty alcohols, C14-22 fatty acids, C23-60 carboxylic acids, hydrogenated vegetable oils, polymers, sorbitan esters and other high molecular weight esters.

The wetting agent can be a surfactant or a mixture of surfactants. The surfactants can be non-ionic surfactants or ionic surfactants. The ionic surfactants can be either positively charged or negatively charged. The examples of non-ionic surfactants include alkyl poly(ethylene oxide) such as copolymers of poly(ethylene oxide) and poly(propylene oxide) (commercially called Poloxamers or Poloxamines), alkyl polyglucosides such as octyl glucoside and decyl maltoside, fatty alcohols such as cetyl alcohol, oleyl alcohol, cocamide MEA and cocamide DEA. The examples of ionic surfactants include anionic (e.g., based on sulfate, sulfonate or carboxylate anions) surfactants such as SDS, ammonium lauryl sulfate and other alkyl sulfate salts, sodium laureth sulfate, also known as sodium lauryl ether sulfate (SLES), Alkyl benzene sulfonate, soaps, or fatty acid salts; and Cationic (e.g., based on quaternary ammonium cations) surfactants such as Cetyl trimethylammonium bromide (CTAB) a.k.a. hexadecyl trimethyl ammonium bromide, and other alkyltrimethylammonium salts, Cetylpyridinium chloride (CPC), Polyethoxylated tallow amine (POEA), Benzalkonium chloride (BAC), Benzethonium chloride (BZT); or Zwitterionic (amphoteric) surfactants such as Dodecyl betaine, Dodecyl dimethylamine oxide, Cocoamidopropyl betaine, Coco ampho glycinate. Alternatively, the wetting agents may also be hydrophilic molecules. The hydrophilic molecules may be small molecules such as sucrose, glucose and glycerol. The hydrophilic molecules may also be polymers such as polyethylene glycol and its copolymers.

### Substrate

In one embodiment of the present invention, the wetness indicator composition of the present invention may be on and/or in a substrate. When present on a substrate, the wetness indicator composition will typically be placed on and/or in a substrate where the substrate will be contacted by a liquid, such as water, urine, menses, blood and the like. The substrate may include, but is not limited to, a structural component, such as woven fabrics, nonwoven fabrics, films, sponges, and combinations thereof. The substrate may comprise synthetic and/or natural materials. In one embodiment of the present invention the optional substrate may be an article in its own right, such as, a continuous nonwoven fabric. In another embodiment of the present invention the substrate to which the wetness indicator composition may be applied or otherwise affixed comprises any one, or a combination of, structural components of an absorbent article, including, but not limited to, the backsheet, topsheet, fasteners, absorbent material, etc., or may be a separate element added or applied to the product. In one embodiment of the present invention the wetness indicator composition is applied to the absorbent article as a whole. In some embodiments, the wetness indicator composition is a single layer. Such a single layer may be applied to a substrate or structural component. In some embodiments, the single-layer formulation may be disposed between the backsheet and the absorbent core, in other embodiments, between the topsheet and the absorbent core.

The wetness indicator composition may be coated over a surface of said substrate as either a) a monochromic color scheme alone, bi-chromic, or multiple colors, b) in various shapes and sizes, c) graphics of patterns or alpha numeric symbols and words, or combinations thereof. The color transition may be from being either a) colored to uncolored, b) uncolored to colored, c) colored to different colored, or d) a combination of a) and b) and c).

The following discussion is for convenience of formulation, but is not intended to limit the type of substrate used herein.

Figure 1 is a plan view of an absorbent article, in this case a diaper 20, of the present invention in a flat, uncontracted state with portions of the structure being cut away to more clearly show the construction of the diaper. The portion of the diaper 20 that faces a wearer is oriented towards the viewer. As shown in Figure 1, the diaper 20 comprises a topsheet 24; an outer cover 26; an acquisition layer (not shown), and an absorbent core 28 that is positioned between at least a portion of the topsheet 24 and the backsheet 26. The absorbent article further comprises side panels 30, elasticized leg cuffs 32, elastic waist features 34, and a fastening system generally designated 40. The diaper 20 has a first waist region 36, a second waist region 38 opposed to the first waist region 36, and a crotch region 37 located between the first waist region 36 and the second waist region 38. The periphery of the diaper 20 is defined by the outer edges of the diaper 20 in which longitudinal edges 50 run generally parallel to a longitudinal centerline 100 of the diaper 20 and end edges 52 run between the longitudinal edges 50 generally parallel to a lateral centerline 110 of the diaper 20.

The outermost surface of the backsheet/outer cover 26 forms the garment contacting surface (not shown) of the diaper 20, while the innermost surface of the topsheet 24 forms the body contacting surface (not shown) of the diaper 20. The absorbent articles of the present invention comprise a topsheet 24. In one example, the topsheet 24 is compliant, soft feeling, and non-irritating to the wearer's skin. It can be elastically stretchable in one or two directions. Further, the topsheet is liquid pervious, permitting liquids (e.g., menses, urine, and/or runny feces) to readily penetrate through its thickness. A suitable topsheet can be manufactured from a wide range of materials such as woven and nonwoven materials; apertured or hydroformed thermoplastic films; porous foams; reticulated foams; reticulated thermoplastic films; and thermoplastic scrims. Suitable woven and nonwoven materials may comprise of natural fibers such as wood or cotton fibers; synthetic fibers such as polyester, polypropylene, or polyethylene fibers; or combinations thereof. If the topsheet includes fibers, the fibers may be spunbond, carded, wet-laid, meltblown, hydroentangled, or otherwise processed as is known in the art.

In one embodiment, the backsheet 26 is impervious to fluids (e.g., menses, urine, and/or runny feces) and is manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core from wetting articles which contact the absorbent article such as bedsheets, pants, pajamas and undergarments. The backsheet 26 may thus comprise a woven or nonwoven material, polymeric films such as thermoplastic films of polyethylene or polypropylene, and/or composite materials such as a film-coated nonwoven material (i.e., having an inner film layer and an outer nonwoven layer). The backsheet 26 and the topsheet 24 are positioned adjacent a garment surface and a body surface, respectively, of the absorbent core 28.

The articles of the present invention additionally comprise one or more absorbent cores 28. The absorbent core 28 is at least partially disposed between the topsheet and the backsheet and may take on any size or shape that is compatible with the disposable absorbent article. The absorbent core 28 may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials (AGM); or any equivalent material or combinations of materials, or mixtures of these. Further useful materials and constructions appropriate for the topsheets, backsheets, outer covers, and absorbent cores described herein may be found in U.S. Ser. No. 14/302,473.

The absorbent core may comprise, consist essentially of, or consist of, a core wrap, absorbent material, and glue enclosed within the core wrap. The absorbent material may comprise superabsorbent polymers, a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core may have areas having little or no absorbent material, where a wearer-facing surface of the core bag may be joined to a garment-facing surface of the core bag. These areas having little or no absorbent material may be referred to as "channels". These channels can embody any suitable shapes and any suitable number of channels may be provided. In other instances, the absorbent core may be embossed to create the impression of channels.

The articles of the present invention may comprise at least one graphic, which refers to images or designs that are constituted by a figure (i.e., a line(s)), a symbol or character, a color difference or transition of at least two colors, or the like. The graphic may have an aesthetic image or design that can provide certain benefits when the absorbent article of the invention is viewed by users or consumers. A variety of graphics can be used in the absorbent articles of the invention.

The article may further comprise at least one wetness indicator 60. A wetness indicator can be located on or against any surface of a component material, including the body contacting surface and the garment contacting surface provided that the wetness indicator 60 remains visible from the exterior of the absorbent article. Non-limiting examples of the component material include the backsheet film/NW, the topsheet, the acquisition layer, the absorbent core, and the barrier leg cuffs. In another embodiment, a wetness indicator 60 is disposed between the absorbent core and the backsheet and in liquid communication with the absorbent core.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

### RHEOLOGY METHOD DETAILS:

The rheometer used for the rheology measurements was a TA Instruments AR-G2 stress controlled rheometer (model #532001.901) equipped with a TA Instruments ETC oven attachment (model # 543401.982). The rheological measurements also used a concentric cylinder peltier jacket (TA Instruments Part #533201.901), a sand blasted aluminum standard concentric cylinder cup (TA Instruments Part #545622.901), a sand blasted aluminum conical DIN rotor (TA Instruments Part #546011.901), and a peltier concentric cylinder solvent trap (TA Instruments Part #545609.901). For controlling the temperature of the sample, the rheometer was connected to a VWR refrigerated water circulator (model #MX7L R-20) which was capable of providing cooling water down to a temperature of 5°C.

The following program settings were used for the Oscillatory Temperature Ramp sequence for acquisition of G' (pascals) and Temperature (degrees Celsius) values.

### Wetness Indicator Profiling 1 v2.0_Osc Techniques Summary -

Instrument Type: TA-Instruments Model ARG2
- Step 1) Oscillation-Temperature Ramp :
   Start temperature 145 °C
   Soak time 300.0 seconds, Wait for temperature: Off
   Ramp rate 2°C/min
   End temperature 5 °C
   Soak time after ramp 0.0 seconds
   Sampling interval 30 seconds per point
   Strain % set to 0.01%
   Single point
   Frequency 0.5Hz
- Step 2) Oscillation-Temperature Ramp :
   Start temperature is 5 °C (Use entered value of 5 °C)
   Soak time 300.0 seconds. Wait for temperature: Off
   Ramp rate 2°C/min
   End temperature 145 °C
   Soak time after ramp 0.0 seconds
   Sampling interval 30 seconds per data point
   Strain % set to 0.01%
   Single point
   Frequency 0.5Hz
- Step 3) Conditioning-End Of Test:
   Set temperature: On Temperature 130 °C
   Set temperature system idle (only if axial force control is active): Off

   Date Run: [Per run date]
   Geometry Name: #545012.001 DIN concentric cylinders, Peltier Aluminium SB (sandblasted)
   TA Instruments Trios Version: 3.3.1.4055

The following program settings were used for the Flow Temperature Ramp and the Shear Sweep sequence for acquisition of shear rate (sec⁻¹) and viscosity (Pa.sec) values.

Wetness Indicator Profiling 2 v1.0_Flow Techniques Summary - Instrument Type: TA Instruments Model ARG2
- Step 1) Conditioning-Sample :
   Temperature 145 °C with Inherit Set Point: Off
   Soak Time 300.0 seconds and Wait For Temperature: On
      Wait for axial force: Off Perform preshear: Off Perform equilibration: Off
- Step 2) Flow-Temperature Ramp :
   Start temperature 145 °C and Use entered value
   Soak time 0.0 seconds and Wait for temperature: Off
   Ramp rate 1.0 °C/min
   End temperature 120 °C
   Soak time after ramp 0.0 seconds
   Shear Rate 101/s
   Sampling interval 10seconds/pt
- Step 3) Flow-Temperature Ramp :
   Start temperature 120 °C and Use entered value
   Soak time 300.0 seconds and Wait for temperature: Off
   Ramp rate 1.0 °C/min
   End temperature 145 °C
   Soak time after ramp 0.0 seconds
   Shear Rate 101/seconds
   Sampling interval 10seconds/point
- Step 4) Flow-Sweep :
   Temperature 120 °C and Inherit Set Point: Off
   Soak Time 300.0 seconds and Wait For Temperature: On
   Logarithmic sweep
   Shear rate 0.01 to 10001/seconds
   Points per decade set to 20
   Steady state sensing: On Max. equilibration time 60.0 seconds
   Sample period 5.0 seconds
   % tolerance 5.0
   Consecutive within 3
   Scaled time average: Off
- Step 5) Conditioning-End Of Test:
   Set temperature: On Temperature set to 135 °C
   Set temperature system idle (only if axial force control is active): Off
   Date Run: [Per run date]
   Geometry Name: #545012.001 DIN concentric cylinders, Peltier Aluminium SB (sandblasted)
   TA Instruments Trios Version: 3.3.1.4055

DATA ANALYSIS PROTOCOLS - used to generate rheological data; using raw data obtained from oscillatory: temp ramp and flow: temperature ramp & shear sweep method sequences.

-The raw data obtained from the testing protocols above were further analyzed in two different ways: Software Analysis (conducted via the Trios software from TA Instruments) or Mathematical Calculations (Microsoft Excel or equivalent software)

Software Analysis: all of the data generated by software analysis was achieved by highlighting the appropriate data range, right clicking on the data graph (set up as described in the previous sections) and choosing analyze -> [selection]

Delta G' - obtained from selecting [Signal Change] on a range of data as displayed in a plot of G' (y-axis) vs. temperature (x-axis) obtained from the Oscillatory temperature ramp sequences (from Wetness Indicator Profiling 1 v2.0_Osc Techniques)

Infinite Shear Rate - obtained from selecting [Cross model] on a range of data as displayed in a plot of viscosity (pas) (y-axis) vs. shear rate (1/s) (x-axis) obtained from the Flow test sequence, shear sweep (from Wetness Indicator Profiling 2 v1.0_Flow Techniques)

Mathematical Calculations: all of the data generated from mathematical calculations were conducted using raw data or software data.

Delta C - value calculated by taking the setpoint or meltpoint temperature in degrees C minus the endpoint temperature in degrees C (setpoint is the first point that appears before the G' increase, meltpoint is the first point that appears before the G' decrease, endpoint is the first point that is stable after the G' increase or decrease) (from Wetness Indicator Profiling 1 v2.0_Osc Techniques)

Delta G'/delta C - value calculated by taking the Delta G' dividing by the Delta C

## Claims

1. An article for baby care or feminine care, said article comprising a wetness indicator comprising:
(a) at least one colorant;
(b) at least one stabilizer;
(c) from0.1% to 70% by weight of at least one of a hardening agent and a crystallizing agent;
(d) wherein the wetness indicator has at least three of the parameters consisting of:
(i) a hot to cold solidification rate of Delta(G')/Delta(°C) from3,800 to27,000 Pa/°C;
(ii) a set point temperature from60°C tol 10°C;
(iii) an infinite shear rate from0.02 to0.5 sec-1;
(iv) a cold to hot melting rate from3,700 to 11,000 Pa/°C; and
(v) a melt point temperature from58°C to 135 °C.

2. The article of claim 1, wherein the wetness indicator has a hardness, expressed as a Needle Penetration measured according to ASTM D1321-04, that is no greater than40 dmm at 23 °C and no greater than150 dmm at 55 °C.

3. The article of any one of the preceding claims, wherein the hardening agent or crystallizing agent comprises a long-chain alkyl chain moiety

4. The article of any one of the preceding claims, wherein the hardening agent or crystallizing agent is selected from the group consisting of long chain linear primary carboxylic acids, polyolefin waxes, paraffin waxes, oxidized polyolefin waxes, maleic anhydride waxes, montan waxes and esters, C14- C50 fatty alcohols, C14 - C50 fatty acids, hydrogenated vegetable oils, semi-crystalline polymers, polyesters, sorbitan esters and other high molecular weight esters, sucrose esters, and combinations thereof.

5. The article of any one of the preceding claims, wherein the hardening agent or crystallizing agent is a long chain, linear primary carboxylic acid.

6. The article of any one of the preceding claims, wherein the wetness indicator comprises at least15% of a hardening agent or crystallizing agent.

7. The article of any one of the preceding claims, wherein the wetness indicator comprises from50% to70% of a hardening agent or crystallizing agent.

8. The article of any one of the preceding claims, wherein the at least one colorant is selected from the group consisting of the free acid of bromophenol blue, the free acid of bromocresol green, the free acid of bromocresol purple and combinations thereof.

9. The article of any one of the preceding claims, further comprising a permanent colorant.

10. The article of any one of the preceding claims, further comprising a hot melt binding matrix.

11. The article of claim 10, wherein the hot melt binding matrix comprises one or more components selected from the group consisting of a binding agent, a tackifier, a surfactant, a structural adjunct, an anti-oxidant, UV stabilizers, plasticizers, and combinations thereof.

12. The article of claim 11, wherein the hot melt binding matrix comprises at least one binding agent selected from the group consisting of acrylic-based binders, amide based binders, amine based binders, adhesives, hot melt adhesive components, waxes and modified waxes like oxidized waxes, surfactants, rosin esters, rosins and polymerized rosins, modified styrene-acrylic polymers and their salts, polyethylene glycols, styrenated terpenes, polyterpene resins, terpene phenolics, quaternary ammonium compounds, quaternary polymers, rubbers, cationic clay materials, ethoxylated quaternary ammonium compounds, quaternized silicone compounds, cationic guars, cationic exchange resins, anionic ingredients like anionic exchange resins, and combinations thereof.

13. The article of claim 10 , wherein the hot melt binding matrix comprises a first binding agent and a second binding agent.

14. The article of any one of the preceding claims, wherein the stabilizer is selected from the group consisting of linear primary carboxylic acids, acidic waxes, acidic phosphate esters, acidic rosin esters, copolymers of ethylene with acrylic or methacrylic acid or combinations thereof.

15. The article of any one of the preceding claims, wherein the wetness indicator comprises at least50% by weight of the hardening agent or crystallizing agent.
